# EUROPEAN PATENT SPECIFICATION

(11) **EP 4 401 704 B1**
(45) Date of publication and mention of the grant of the patent: **16.07.2025**
(21) Application number: 22772918.3
(22) Date of filing: 01.09.2022
(51) Int. Cl.: A61K 8/891, A61K 8/892, A61Q 1/00, A61Q 1/02

(54) **CARE AND/OR MAKEUP COMPOSITION COMPRISING A SILICONE ELASTOMER CONTAINING CARBOXYLIC ACID FUNCTIONS AND A COPOLYMER BASED ON SILICONE RESIN AND ON SILICONE OF DIMETHICONOL TYPE**
PFLEGE- UND/ODER SCHMINKZUSAMMENSETZUNG MIT EINEM SILIKONELASTOMER MIT CARBONSÄUREFUNKTIONEN UND EINEM COPOLYMER AUF BASIS VON SILIKONHARZ UND DIMETHICONOLSILIKON
COMPOSITION DE SOIN ET/OU DE MAQUILLAGE COMPRENANT UN ÉLASTOMÈRE SILICONE CONTENANT DES FONCTIONS ACIDE CARBOXYLIQUE ET UN COPOLYMÈRE À BASE DE RÉSINE DE SILICONE ET DE SILICONE DE TYPE DIMÉTHICONOL

(30) Priority: 15.09.2021 FR 2109681
(43) Date of publication of application: 24.07.2024
(73) Proprietor: L'OREAL, 75008 Paris (FR)
(72) Inventor: KERGOSIEN, Guillaume, 94152 CHEVILLY LARUE (FR); DU, Kévin, 94152 CHEVILLY LARUE (FR)
(74) Representative: L'Oreal
(86) International application number: PCT/EP2022/074346
(87) International publication number: WO 2023/041343

(56) References cited:
- WO-A1-2015/066161
- WO-A1-2015/066199
- US-A- 5 162 410
- "MAKE-UP AND SKIN CARE COMPOSITIONS CONTAINING A SPECIFIC SILICON GEL ED - Darl Kuhn", IP.COM, IP.COM INC., WEST HENRIETTA, NY, US, 20 June 2016 (2016-06-20), XP013171967, ISSN: 1533-0001

## Description

### Technical field

The present invention aims to provide, for the field of caring for and/or making up keratin materials, in particular the skin, a new composition comprising at least one silicone elastomer containing carboxylic acid functions, and at least one copolymer based on silicone resin and on silicone of dimethiconol type.

Cosmetic makeup compositions are commonly used to give keratin materials such as the skin an attractive colour, but also to enhance the beauty of uneven skin, by making it possible to hide marks and dyschromias, and to reduce the visibility of relief imperfections such as pores and wrinkles. Many formulations have been developed to date. For several years, consumers have been searching for compositions which provide a good wear property of the makeup effect over time in order to be able to avoid having to reapply the composition too often.

One of the solutions proposed by the prior art for obtaining a good wear property is the use of a film-forming agent which allows the composition, once applied, to form, after drying, a more cohesive, adhesive and persistent film on the support. However, the use of film-forming agents is not entirely satisfactory since one of the problems encountered with such agents lies in the fact that they cause discomfort when used, produced notably by difficulty in spreading the composition.

Makeup compositions based on silicone elastomers containing carboxylic acid functions have already been proposed in the published documents WO2015066161 (US20160200876), WO2015066165, WO2015066199, WO2015167963, WO20180165434 (US10918587B2), IPCOM000250448D, IPCOM000250657D, which compositions have good performance in terms of wear property and durability. However, these polymers are not completely satisfactory in terms of resistance to friction. Furthermore, the silicone elastomers containing carboxylic acid functions generally have a texture that is difficult to take up and apply and have a tendency to give makeup compositions where the insufficiently fluid, supple and homogeneous consistency does not enable the product to be taken up and easily spread on the skin.

One of the objectives of this invention is to produce a composition for caring for and/or making up keratin materials that comprises at least one silicone elastomer containing carboxylic acid functions in order to obtain a formula having a deposit that is substantially more resistant to friction.

Another objective of this invention is to produce a composition for caring for and/or making up keratin materials that comprises at least one silicone elastomer containing carboxylic acid functions that has a sufficiently fluid, smooth, supple and homogeneous consistency to be taken up and easily spread on the skin.

During its research studies, the applicant has discovered, unexpectedly, that these objectives could be achieved with a composition for caring for and/or making up keratin materials, notably the skin, comprising, preferably in a physiologically acceptable medium:
a) at least one silicone elastomer containing carboxylic acid functions; and
b) at least one copolymer based on silicone resin and on silicone of dimethiconol type.

This discovery forms the basis of the invention.

The present invention relates to a composition for caring for and/or making up keratin materials, in particular the skin, comprising, preferably in a physiologically acceptable medium:
a) at least one silicone elastomer containing carboxylic acid functions; and
b) at least one copolymer based on silicone resin and on silicone of dimethiconol type.

According to a preferred embodiment, the composition is anhydrous.

The invention also relates to a process for coating keratin materials, more particularly for making up and/or caring for keratin materials, such as the skin, characterized in that it comprises the application to keratin materials of an emulsion as defined above.

The invention more particularly relates to a process for making up and/or caring for the skin, characterized in that it comprises the application to the skin of a composition as defined above.

### Definitions

In the context of the present invention, the term "keratin materials" means the skin and more particularly the areas such as the face, the cheeks, the hands, the body, the legs and thighs, the area around the eyes, and the eyelids.

The term "physiologically acceptable" means compatible with the skin and/or its integuments, which has a pleasant colour, odour and feel, and which does not cause any unacceptable discomfort (stinging, tautness or redness) liable to discourage the consumer from using this composition.

For the purposes of the present invention, the term "anhydrous" means a composition comprising a content of less than or equal to 2% by weight of water, preferably less than or equal to 1%, more preferentially less than 0.5% by weight relative to the total weight of said composition, or is even free of water. Where appropriate, such small amounts of water may notably be introduced by ingredients of the composition that may contain residual amounts thereof.

### Silicone elastomer containing carboxylic acid functions

The composition according to the invention the caring for and/or making up keratin materials, comprises at least one silicone elastomer containing carboxylic acid functions.

The term "organopolysiloxane elastomer" means a supple, deformable organopolysiloxane that has viscoelastic properties and especially the consistency of a sponge or a supple sphere. Its modulus of elasticity is such that this material withstands deformation and has limited stretchability and contractibility. This material is capable of regaining its original shape after stretching. In general, it is partially or completely crosslinked and is not cyclic.

The term "silicone elastomer containing carboxylic acid functions" means an organopolysiloxane elastomer comprising in its structure at least two carboxylic acid groups with at least one -COOH function.

The composition according to the invention advantageously has a content of active material of silicone elastomer containing carboxylic acid functions ranging from 2% to 25% by weight relative to the weight of the composition. Preferably, this content ranges from 5% to 15% by weight relative to the weight of the composition.

Silicone elastomers containing carboxylic acid functions that can be used in the compositions of the invention can be chosen from those described in the published documents WO2015066161 (US20160200876), WO2015066165, WO2015066199, WO2015167963, WO20180165434 (US10918587B2), IPCOM000250448D, IPCOM000250657D.

According to one particular embodiment, the silicone elastomer containing carboxylic acid functions is chosen from those corresponding to formula (I) below: 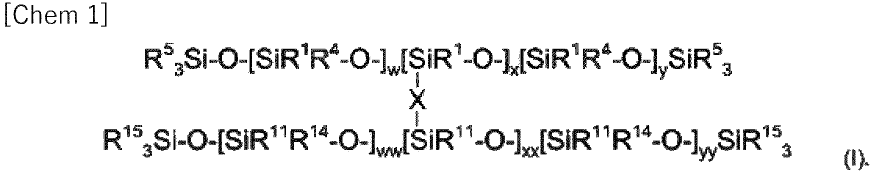 in which:
the radical X corresponds to formula (i) below 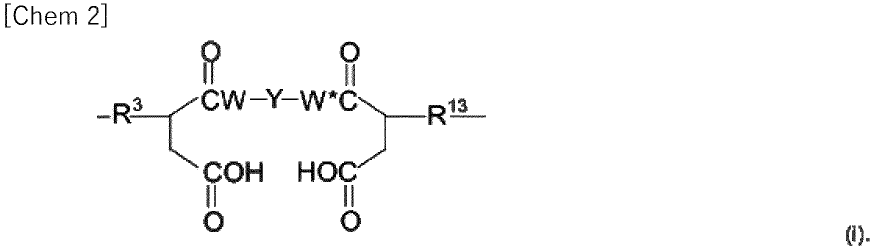 or to the formula (i') below 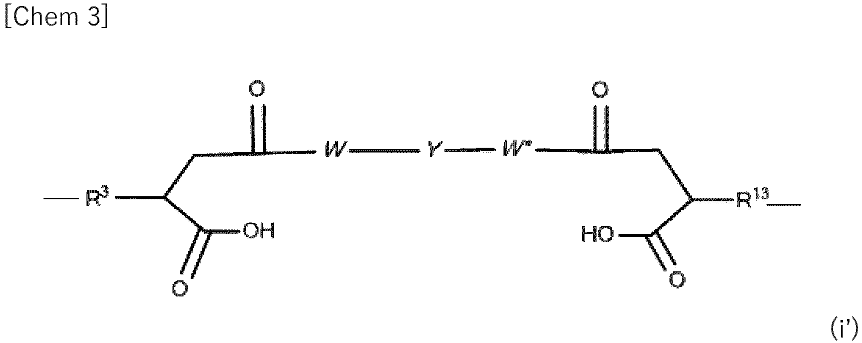 in which:
W and W* independently denote an oxygen atom (O) or an N-R group where each R radical independently denotes a hydrogen atom (H) or an R1 radical;
Y is a divalent group;
R¹, R¹¹, R⁴, R¹⁴, R⁵ and R¹⁵ independently denote a substituted or unsubstituted hydrocarbyl group;
R³ and R¹³ independently denote a divalent group;
w and ww are, independently, integers ranging from 0 to 1000;
x and xx are, independently, integers ranging from 1 to 100;
y and yy are, independently, integers ranging from 0 to 1000;
preferably, w and y are not simultaneously equal to 0 and ww and yy are not simultaneously equal to 0.

The term "substituted" means one or more hydrogen atoms of the hydrocarbyl group are replaced by an atom other than hydrogen (i.e. halogen) or else that one or more carbon atoms are replaced by an atom other than carbon such as a heteroatom such as oxygen, sulfur or nitrogen.

In formula (I), the lower and upper portions are identical or different siloxanes. According to certain specific embodiments, each siloxane chain comprises siloxane bonds (Si-O-Si) in their respective backbone. Each siloxane chain may include siloxane bonds separated by one or more divalent groups, for example a -CH₂- linking group. Other examples of divalent groups may include polyether groups: for example - CH₂CH₂O- (i.e. ethylene oxide group), -CH(CH₃)CH₂O- (i.e. propylene oxide), etc. linking groups. Combinations of different divalent groups may be present in their respective backbone. Each of the divalent groups may be singular or repeated, i.e.: 2 times, 5 times, 10 times, or even > 10 times, etc. According to certain embodiments, the siloxanes do not contain any polyether groups.

In various embodiments, one or both siloxanes may comprise at least one [SiR¹R²-O-] unit ("D" or R*₂SiO_{2/2} units). Typically, each siloxane has a repetition of D units, which generally constitutes the linear portions of the siloxanes. The siloxanes also typically have terminal R*₃SiO_{1,2} units ("M" units).

In certain specific embodiments, one of the two siloxanes or both siloxanes may optionally be branched, partially branched and/or include a resin portion having a three-dimensional structure network. In such cases, the corresponding siloxane(s) may additionally comprise R*SiO_{3/2}units ("T" units) and/or SiO_{4/2}units ("Q" units). The branched or resinous nature of the siloxane(s) may be attributed to the presence of T and/or Q units. The branched nature of a siloxane may be attributed to side groups of one or more D units. According to specific embodiments, one of the siloxanes or both siloxanes may be devoid of T units and/or of Q units. The two siloxanes may be identical or different: i.e. one is linear and the other is branched, or else both siloxanes are linear.

According to certain specific embodiments, the R¹ radicals independently denote an alkyl, aryl, alkenyl, alkaryl, or aralkyl radical. More particularly, the R¹ radicals denote an alkyl radical, preferably having from 1 to 20, from 1 to 15, from 1 to 10, from 1 to 6, from 1 to 4, or 1 or 2 carbon atoms such as for example methyl, ethyl, propyl, butyl, pentyl. More particularly, all the R¹ radicals denote a methyl group (i.e. -CH₃).

Preferably, the R³ radical is a hydrocarbylene, heterohydrocarbylene, or organoheterylene group. In particular, R³ is a (CH₂)ₙ group where n is an integer ranging from 1 to 30, from 1 to 25, from 1 to 20, from 1 to 15, from 1 to 10, from 1 to 5, or from 1 to 3, and more particularly equal to 3.

According to various specific embodiments, each of the R⁴ and R³ radicals may independently denote a substituted or unsubstituted hydrocarbyl group, notably chosen by those defined above for the R¹ radicals.

According to certain embodiments, each R⁴ radical independently denotes an alkyl, aryl (i.e. phenyl) group or a (R⁶O)ₘ group. When R⁴ is a (R⁶O)ₘ group, R⁶ is, preferably, an alkyl or aryl (i.e.: phenyl) radical and m is an integer ranging from 1 to 50, from 1 to 25, from 1 to 10, from 1 to 5, or 1. The (R⁶O)ₘ group may be a polyether group (i.e.: with ethylene oxide and/or propylene oxide units). According to specific embodiments, an R⁴ radical may be a divalent group of a silicone side chain of the siloxane.

According to a specific embodiment, R⁴ may denote an anhydride group of formula (1) 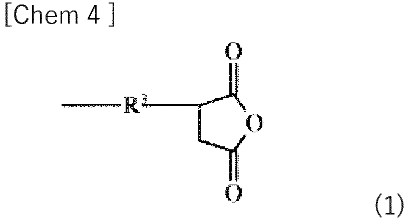 in which R³ has the same definition indicated previously.

Preferably, the R⁴ radicals denote an alkyl radical or a polyether group.

Preferably, each R⁵ radical is an R¹ radical. For example, each R⁵ and R¹ radical is an alkyl group: i.e. methyl.

Preferably, R¹¹ is identical to or different from R¹, R¹⁴ is identical to or different from R⁴, R¹⁵ is identical to or different from R⁵. Preferentially, the R⁵ radicals denote R¹ and/or the R¹⁵ radicals denote R¹¹.

Preferably, w is an integer ranging from 0 to 1000, from 0 to 950, from 0 to 750, from 0 to 500, from 0 to 400, from 1 to 350, from 1 to 300, from 25 to 250, from 50 to 200, from 50 to 150, from 75 to 125, from 90 to 110, from 90 to 100, from 90 to 95, and more particularly is equal to 93.

Preferably, x is an integer from 1 to 100, from 1 to 75, from 1 to 50, from 1 to 25, from 1 to 20, from 1 to 10, or from 1 to 5, and more particularly equal to 3.

Preferably, y is an integer ranging from 0 to 1000, from 0 to 950, from 0 to 750, from 0 to 500, from 0 to 400, from 1 to 350, from 1 to 300, from 1 to 250, from 1 to 200, from 1 to 150, from 1 to 100, from 1 to 75, from 1 to 50, from 1 to 25, from 1 to 20, from 1 to 15, from 1 to 10 or from 1 to 5.

According to various embodiments, w and y are not simultaneously equal to 0. In certain embodiments, the sum w+x+y varies from 25 to 1500, from 25 to 1000, from 25 to 900, from 25 to 800, from 25 to 700, from 25 to 600, from 25 to 500, from 25 to 400, from 25 to 300, from 50 to 200, from 75 to 150, from 85 to 125 or from 90 to 110.

In certain embodiments, x is at least equal to 1, at least equal to 10, at least equal to 25, at least equal to 50, at least equal to 75 or at least equal to 5.

According to specific embodiments, ww may be identical to or different from w, xx may be identical to or different from x and yy may be identical to or different from y. The units of formula (I), notably those represented between brackets with the subscripts w, ww, x, xx, y or yy maybe present in any order, randomly or in block form.

According to certain specific embodiments, the R¹¹ radicals, which may be identical or different, denote a linear, branched or cyclic C₁-C₂₀, C₁-C₁₃, C₁-C₁₀, C₁-C₆, C₁-C₄ or C₁-C₂ alkyl radical, such as methyl, ethyl, propyl, butyl, pentyl, etc. groups. More preferentially, the R¹¹ radicals are a methyl group.

According to certain specific embodiments, the R¹⁴ radicals, which may be identical or different, denote an alkyl or aryl (i.e. phenyl) radical or (R¹⁶O)ₘₘ. When R¹⁴ is an (R¹⁶O)ₘₘ group, R¹⁶ denotes an alkyl or aryl (i.e. phenyl) radical and mm is an integer ranging from 1 to 50, 1 to 25, 1 to 10, 1 to 5, or 1. The (R¹⁶O)ₘₘ group may be a polyether group (i.e.: with ethylene oxide and/or propylene oxide units).

According to specific embodiments, the R¹⁴ radicals denote, independently, an alkyl radical having from 2 to 20, 2 to 15, 2 to10, 2 to 5, or 2 carbon atoms.

According to other specific embodiments, R¹⁴ may be a divalent bonding group of a silicone side chain of the siloxane.

According to a specific embodiment, R¹⁴ may denote an anhydride group of formula (1) 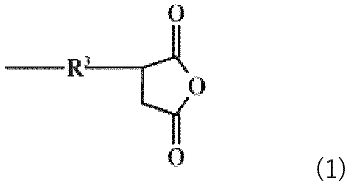 in which R³ has the same definition indicated previously.

Preferably, the R¹⁴ radicals denote an alkyl radical or a polyether group.

Preferably, ww is an integer ranging from 0 to 1000, from 0 to 950, from 0 to 750, from 0 to 500, from 0 to 400, from 1 to 350, from 1 to 300, from 25 to 250, from 50 to 200, from 50 to 150, from 75 to 125, from 90 to 110, from 90 to 100, from 90 to 95, and more particularly 93.

Preferably, xx is an integer ranging from 1 to 100, from 1 to 75, from 1 to 50, from 1 to 25, from 1 to 20, from 1 to 10, or from 1 to 5, and more particularly 3.

Preferably, yy is an integer ranging from 0 to 1000, from 0 to 950, from 0 to 750, from 0 to 500, from 0 to 400, from 1 to 350, from 1 to 300, from 1 to 250, from 1 to 200, from 1 to 150, from 1 to 100, from 1 to 75, from 1 to 50, from 1 to 25, from 1 to 20, from 1 to 15, from 1 to 10 or from 1 to 5.

In various embodiments, ww and yy are not simultaneously equal to 0. According to certain specific embodiments, the sum ww+xx+yy varies from 25 to 1500, from 25 to 1000, from 25 to 900, from 25 to 800, from 25 to 700, from 25 to 600, from 25 to 500, 25 to 400, from 25 to 300, from 50 to 200, from 75 to 150, from 85 to 125, or from 90 to 110.

According to certain specific embodiments, xx is at least equal to 1, at least equal to 10, at least equal to 25, at least equal to 50, at least equal to 75 or at least equal to 85.

The middle portion X of formula (I) corresponds to the general formula (i) below 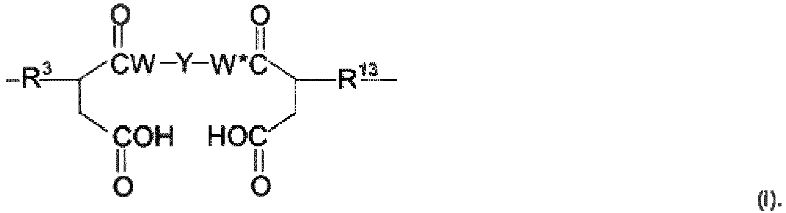 or to the formula (i') below : 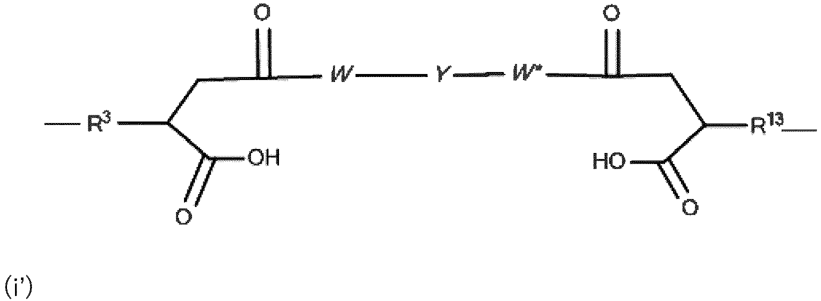 in which:
- each W and W* group denotes, independently, an oxygen atom (O) or an N-R group, with R denoting a hydrogen atom (H) or an R1 radical; preferably R is H;
- R¹³ is a divalent group, preferably is a hydrocarbylene, a heterohydrocarbylene or an organoheterylene; in particular, R13 denotes a (CH₂)ₙₙ radical where nn is an integer from 1 to 30, from 1 to 20, from 1 to 15, from 1 to 10, from 1 to 5, from 1 to 3, and more particularly 3; it being possible for R¹³ to be identical or different relative to R3;
- each Y radical is a divalent group, notably denotes a hydrocarbylene, a heterohydrocarbylene or an organoheterylene radical; in particular, Y is a hydrocarbylene group having from 1 to 50, from 1 to 40, from 1 to 20, from 1 to 10, from 1 to 5, 1 or 2 carbon atoms or may denote a group of formula (ii) below:
   [Chem 5]

   **--[Z]_{d}-[SiR⁸R¹⁰-O-]ₐ[SiR⁸R⁹-O-]_{b}[SiR⁸R¹⁰-O-]_{c}Si-[Z]_{d}-** (II).

   in which:
   - each R⁸, R⁹ denote, independently, a substituted or unsubstituted hydrocarbyl group, notably chosen from an alkyl group (i.e. methyl), an aryl group (i.e. phenyl), a polyether group (i.e. with ethylene oxide and/or propylene oxide units);
   - each R¹⁰ radical denotes, independently, a substituted or unsubstituted hydrocarbyl group such as the radicals defined previously for R¹ and R⁴; in particular, R¹⁰ may be an alkyl group having from 1 to 20 carbon atoms or a polyether group or a divalent linking group of a silicone side chain of the siloxane;
   - each Z radical independently comprises at least one hydrocarbylene, heterohydrocarbylene or organoheterylene group, in particular is a hydrocarbylene group having from 1 to 20, from 1 to 10, from 1 to 5 or 1 carbon atom(s); in particular Z may include siloxane bonds separated by one or more divalent groups, for example a -CH₂- linking group; other examples of divalent groups may include polyether groups: for example - CH₂CH₂O- (i.e. ethylene oxide group),
   - CH(CH₃)CH₂O- (i.e. propylene oxide) linking groups;
   - a is an integer ranging from 0 to 1000, from 0 to 950, from 0 to 750, from 0 to 500, from 0 to 400, from 0 to 300, from 0 to 200, from 0 to 100, from 0 to 75, from 0 to 50, from 0 to 25, from 0 to 20, or -b is an integer ranging from 1 to 1000, from 1 to 950, from 1 to 750, from 1 to 500, from 1 to 400, from 1 to 300 or from 1 to 200;
   - c is an integer ranging from 0 to 1000, from 0 to 950, from 0 to 750, from 0 to 500, from 0 to 400, from 0 to 300, from 0 to 200, from 0 to 100;
   - each d is equal to 0 or 1. In particular, at least one of the d is equal to 1 or both are equal to 1;
   the units of formula (ii) represented between brackets with the subscripts a, b or c being present in any order, randomly or in block form.

According to a specific form, the siloxane of formula (ii) may be a resin of formula R*ₛSiO_{(4-s)/2}. Preferably, a silicone resin has, along its chain, T and/or Q units with M units and optionally D units. The R* radicals may be chosen independently from a substituted hydrocarbonyl group (i.e.: hydroxyl, amine functions) or an unsubstituted hydrocarbonyl group and s varies from 0 to 3. The R* groups that can be used may be those described previously for the R⁸, R⁹, and R¹⁰ radicals. Various combinations of such groups may be present. According to these particular cases, the silicone resin comprises a combination of M, D, T and/or Q units. It may notably be a MDT resin, a MT resin, a MDQ resin, a MQ resin or a MDTQ resin. Each of the M, D, and T units can contain different R* groups. The resin can have various molecular weights, such as a number-average molecular weight ranging from 800 to 500 000.

According to a specific form of the invention, the silicone elastomers containing carboxylic acid functions have a carboxyl equivalent ranging from 100 to 50 000, from 500 to 10 000, or 500 to 5000.

The silicone elastomers containing carboxylic functions of formula (I) may be obtained according to one of the methods of synthesis described in document WO2015066161 (US20160200876).

According to a first variant of the method of synthesis, certain silicone elastomers containing carboxylic acid functions may be obtained by reaction 1) of a first siloxane bearing pendent anhydride groups, 2) of a second siloxane bearing pendent anhydride groups and 3) of an organic polyol. According to a specific embodiment, the 3 preceding reactants can be reacted with one or more siloxanes different from the first two.

According to a second variant of the method of synthesis, certain silicone elastomers containing carboxylic acid functions may be obtained by reaction 1) of a first siloxane bearing pendent anhydride groups, 2) of a second siloxane bearing pendent anhydride groups and 3) of an organic polyamine. According to a specific embodiment, the 3 preceding reactants can be reacted with one or more additional siloxanes different from the first two.

According to a third variant of the method of synthesis, certain silicone elastomers containing carboxylic acid functions may be obtained by reaction 1) of a first siloxane bearing pendent anhydride groups, 2) of a second siloxane bearing pendent anhydride groups and 3) of a third siloxane having at least two hydroxyl groups. According to a specific embodiment, the 3 preceding reactants can be reacted with one or more additional siloxanes different from the first two.

According to a fourth variant of the method of synthesis, certain silicone elastomers containing carboxylic acid functions may be obtained by reaction 1) of a first siloxane bearing pendent anhydride groups, 2) of a second siloxane bearing pendent anhydride groups and 3) of a third siloxane having pendent amine groups and/or terminal amine groups. According to a specific embodiment, the 3 preceding reactants can be reacted with one or more additional siloxanes different from the first two.

According to a fifth variant of the method of synthesis, certain silicone elastomers containing carboxylic acid functions may be obtained by reaction 1) of a first siloxane bearing pendent hydroxyl groups, 2) of a second siloxane bearing pendent hydroxyl groups and 3) of a third siloxane having at least two terminal anhydride groups. According to a specific embodiment, the 3 preceding reactants can be reacted with one or more additional siloxanes different from the first two.

According to a sixth variant of the method of synthesis, certain silicone elastomers containing carboxylic acid functions may be obtained by reaction 1) of a first siloxane bearing pendent amine groups, 2) of a second siloxane bearing pendent amine groups and 3) of a third siloxane having at least two terminal anhydride groups. According to a specific embodiment, the 3 preceding reactants can be reacted with one or more additional siloxanes different from the first two.

According to a seventh variant of the method of synthesis, certain silicone elastomers containing carboxylic acid functions may be obtained by reaction 1) of a first organic alcohol, 2) of a second organic alcohol and 3) of a siloxane having terminal anhydride groups. According to a specific embodiment, the 3 preceding reactants can be reacted with one or more additional organic alcohols different from the first two.

Among the silicone elastomers containing carboxylic acid functions of formula (I), mention may be made of compounds 1 to 12 below as described respectively in examples 4, 5 and 7 to 16 of application WO2015066161:

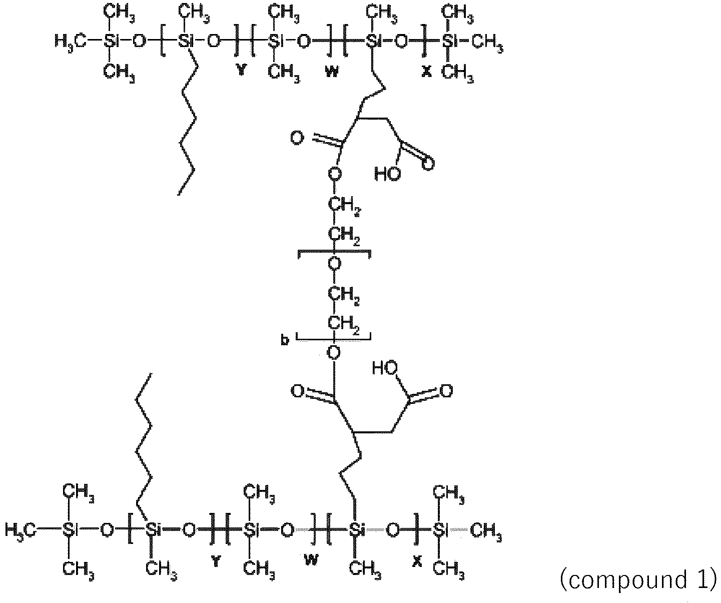

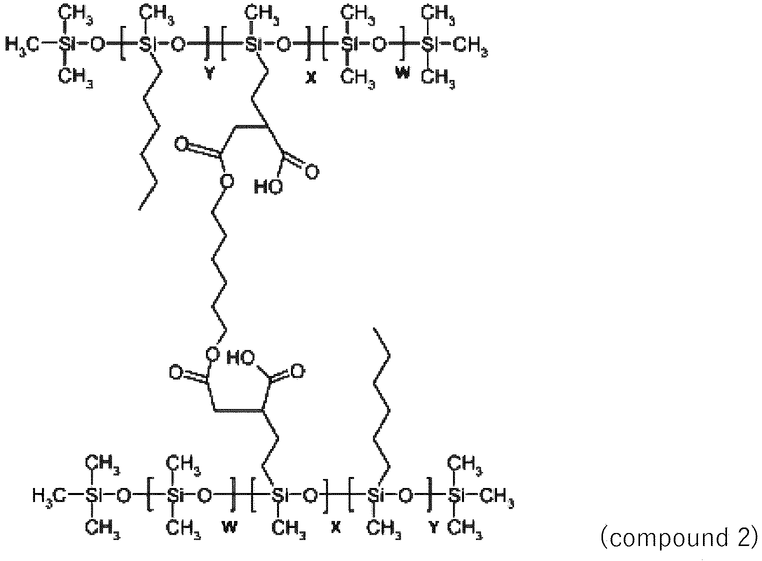

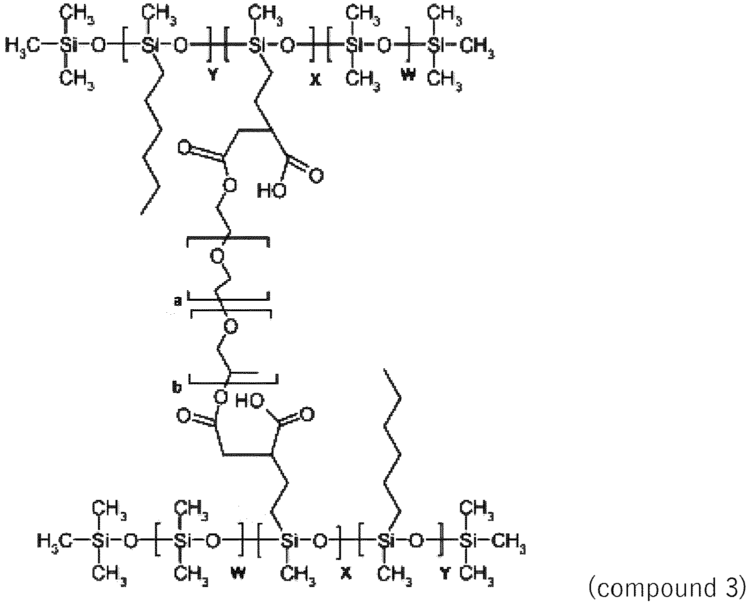

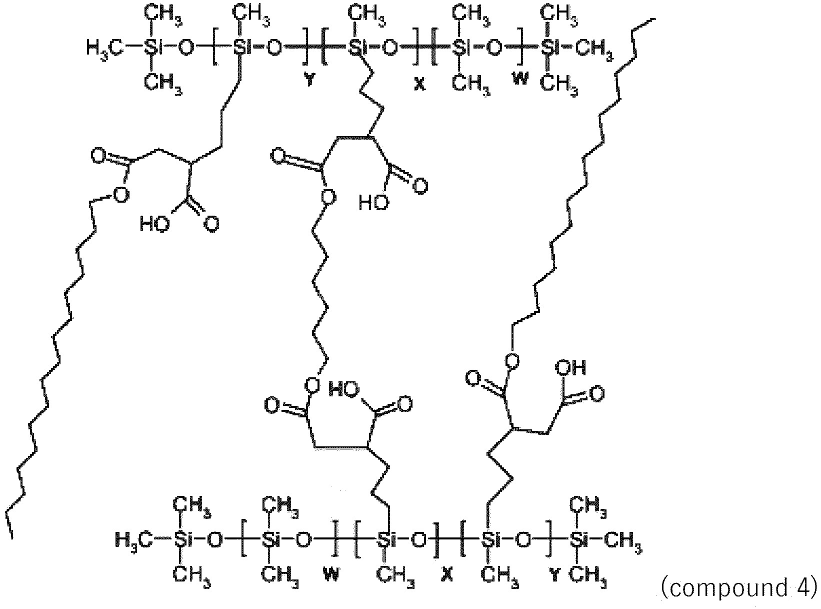

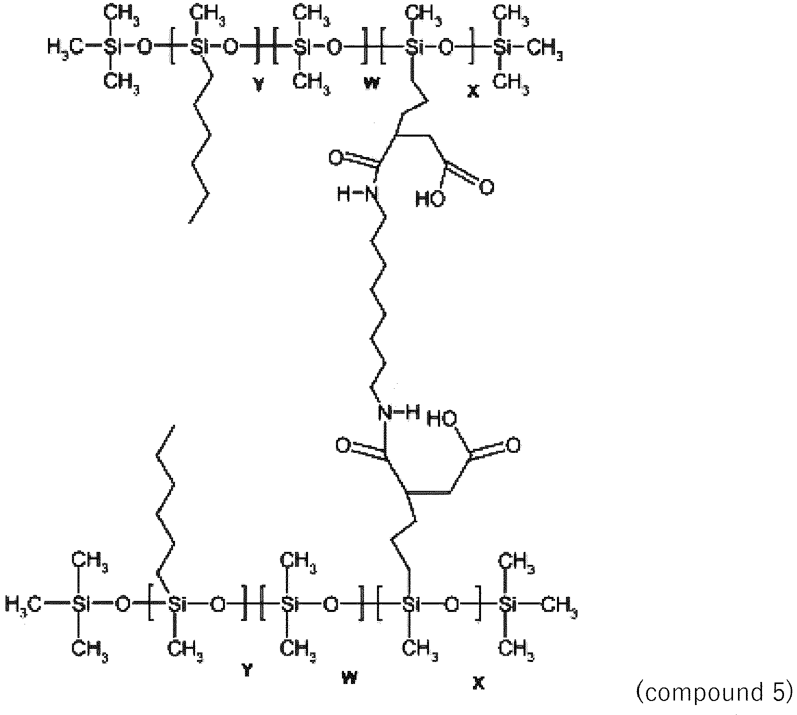

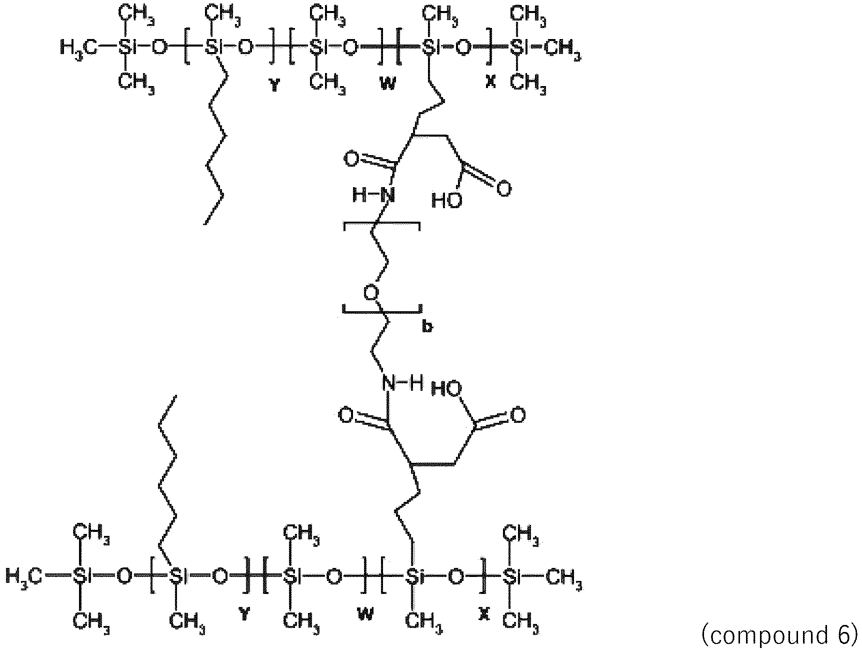

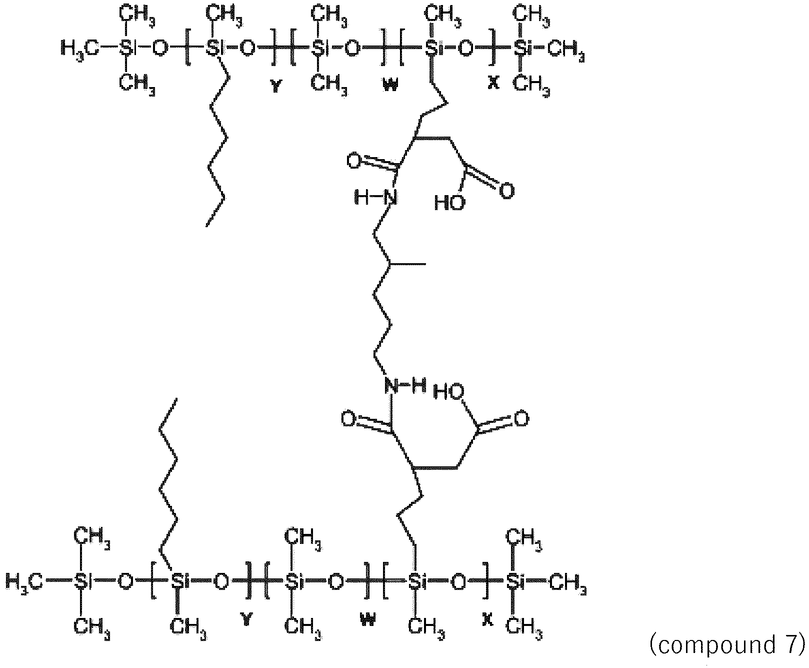

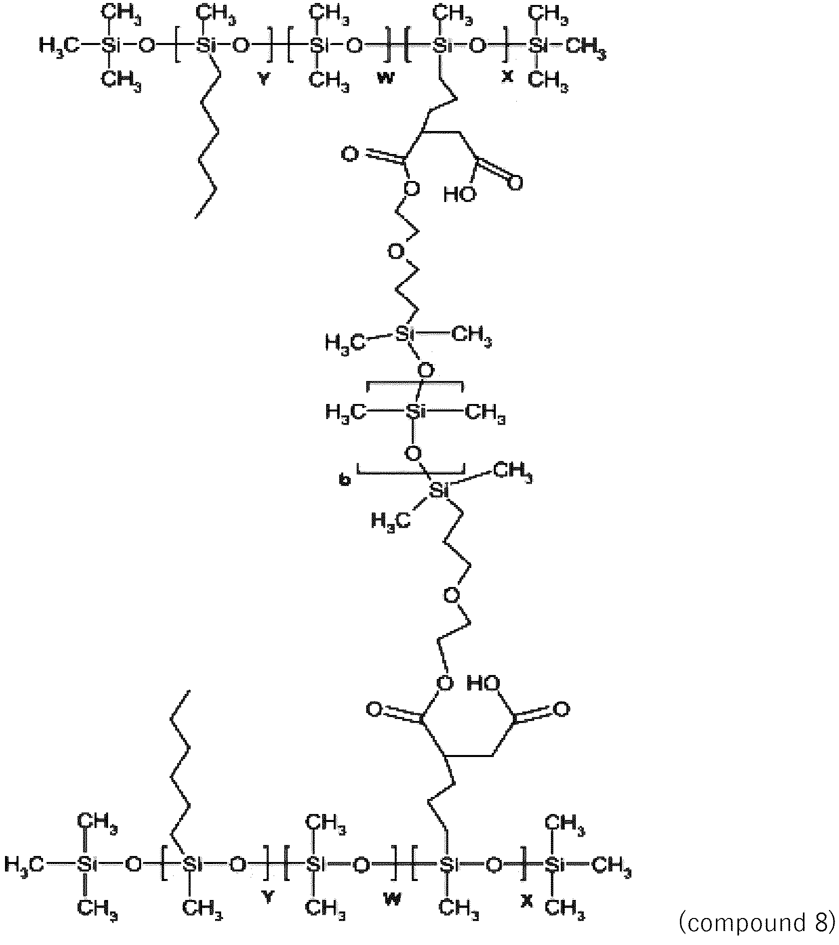

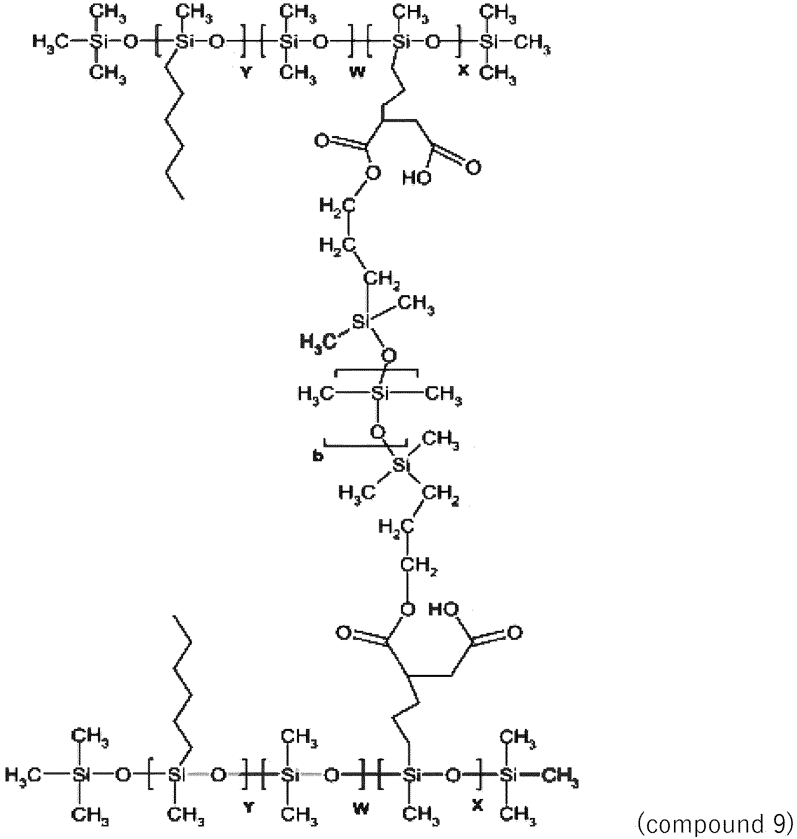

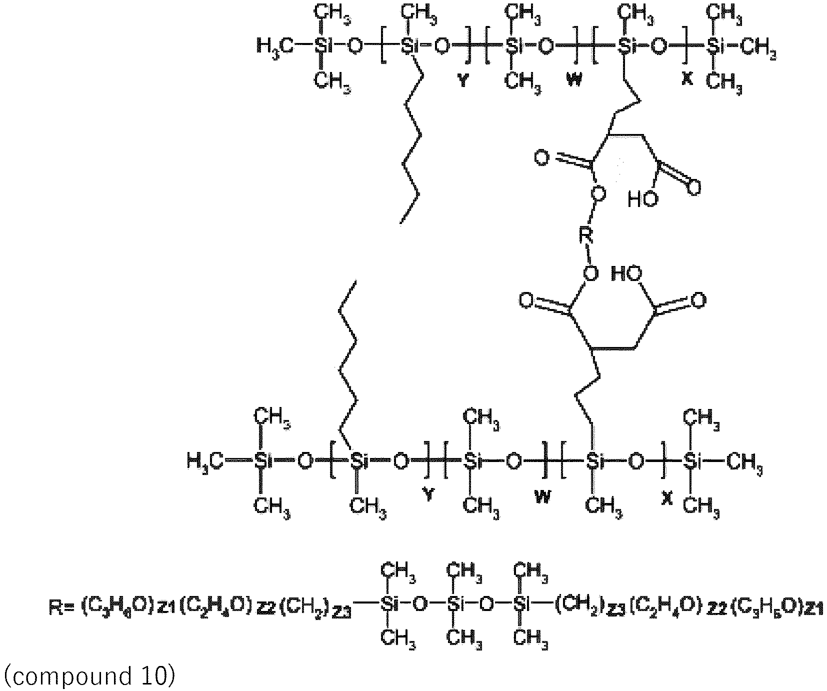

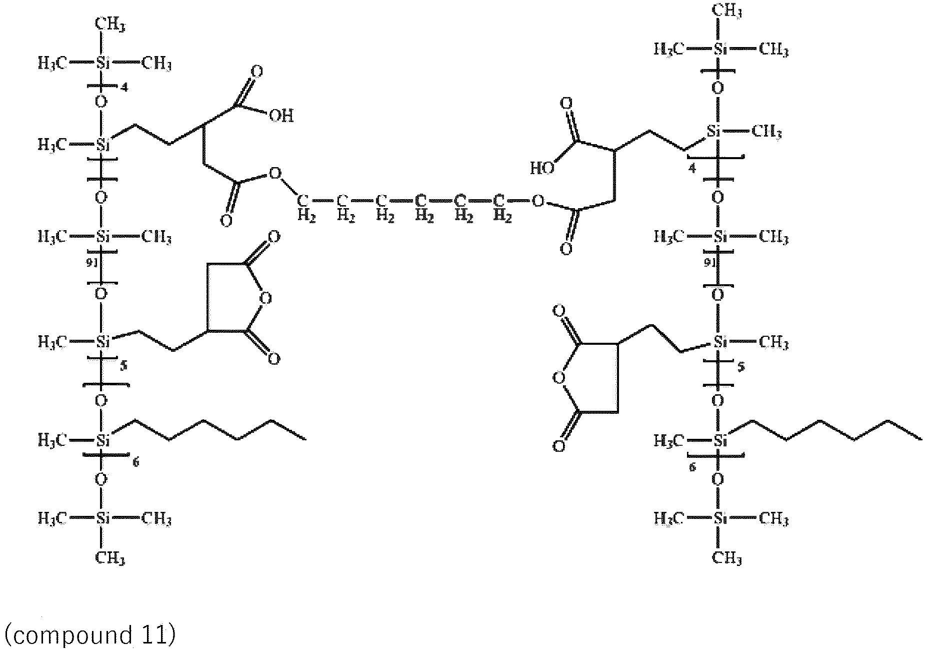

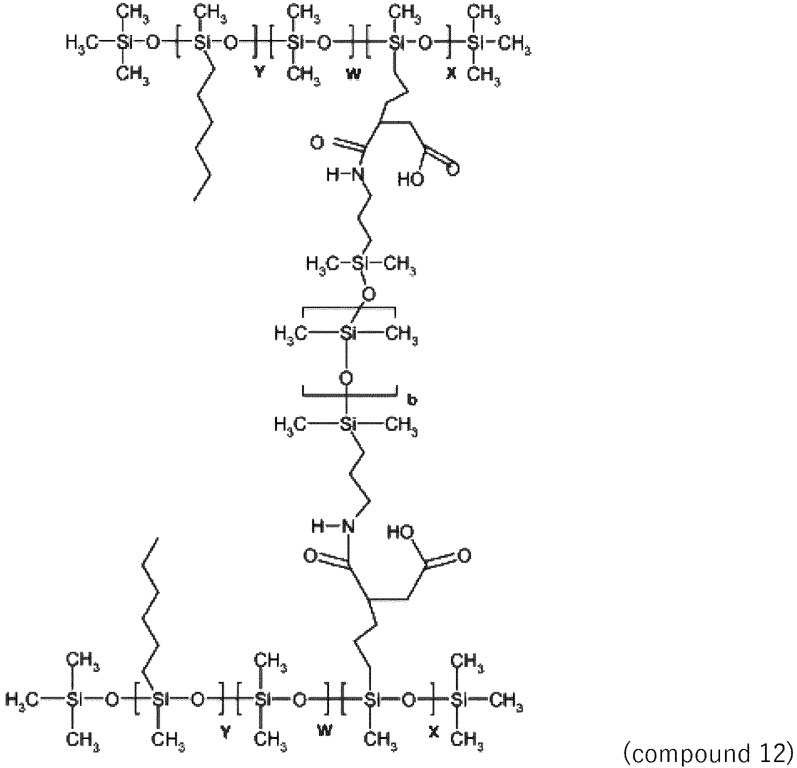

According to a specific form, the composition of the invention contains at least the compound 11 as defined previously.

Among the silicone elastomers containing carboxylic acid functions that can be used in the compositions of the invention, mention may also be made of the silicone elastomer with the INCI name: HEXYL/SUCCINYL DIMETHICONE CROSSPOLYMER.

According to a specific form, the silicone elastomers containing carboxylic acid functions are in solid form. They may notably be chosen from compounds 2 and 3 as defined previously.

According to another specific form, the silicone elastomers containing carboxylic acid functions are in dispersion in at least one oil and are in the form of gel.

The term "oil" is understood to mean a fatty substance which is liquid at ambient temperature (25° C) and atmospheric pressure (760 mmHg, i.e. 10⁵ Pa).

The oil may be chosen from volatile oils and/or nonvolatile oils.

The term "nonvolatile oil" is understood to mean an oil which remains on the keratin material at ambient temperature (25° C) and atmospheric pressure (760 mmHg) for at least several hours and which has in particular a vapour pressure of less than 10⁻³ mmHg (0.13 Pa).

The term «volatile oil" is understood to mean an oil which is able to be evaporated into contact of the skin in less than one hour at room temperature and atmospheric pressure. A volatile oil is a liquid volatile cosmetic at room temperature and atmospheric pressure, notably having a non zero vapor pressure at room temperature and atmospheric pressure, notably having a vapor pressure from 2.66 Pa à 40000 Pa, and particularly from 2.66 Pa à 13000 Pa, and more particularly from 2;66 Pa à 1300 Pa.

The oil may be chosen from hydrocarbon oils, silicone oils, and mixtures thereof.

For the purposes of the present invention, the term "silicone oil" is understood to mean an oil comprising at least one silicon atom and in particular at least one Si-O group, and more particularly an organopolysiloxane.

The term "hydrocarbon oil" is understood to mean an oil comprising mainly carbon and hydrogen atoms and optionally one or more functions chosen from hydroxyl, ester, ether or carboxylic functions.

Mention may be made, as examples of volatile hydrocarbon oil which can be used in the oily dispersion of silicone elastomer containing carboxylic acid functions, of hydrocarbon oils having from 8 to 16 carbon atoms, and in particular C₈-C₁₆ isoalkanes of petroleum origin (also known as isoparaffins), such as isododecane (also known as 2,2,4,4,6-pentamethylheptane), isodecane and isohexadecane, and for example the oils sold under the trade names Isopar or Permethyl, branched C₈-C₁₆ esters, isohexyl neopentanoate and mixtures thereof. Other volatile hydrocarbon oils, such as petroleum distillates, in particular those sold under the name Shell Solt by Shell, can also be used; volatile linear alkanes, such as those described in the patent application DE10 2008 012 457 from Cognis. Use will more particularly be made of isododecane.

Mention may be made, among the volatile silicone oils which can be used in the compositions, for example, of volatile linear or cyclic silicone oils, in particular those having a viscosity of less than or equal to 8 centistokes (8 × 10⁻⁶ m²/s) and having in particular from 2 to 7 silicon atoms, these silicones optionally comprising alkyl or alkoxy groups having from 1 to 10 carbon atoms.

Mention may be made, as volatile linear silicone oil which can be used in the invention, of octamethyltrisiloxane, decamethyltetrasiloxane, dodecamethylpentasiloxane and mixtures thereof.

Mention may be made, as volatile cyclic silicone oil which can be used in the invention, of cyclomethicones such as octamethylcyclotetrasiloxane, decamethylcyclopentasiloxane, dodecamethylcyclohexasiloxane and mixtures thereof.

Mention may be made, as examples of nonvolatile linear silicone oil, of polydimethylsiloxanes (also known as dimethicones); alkyl dimethicones; vinylmethylmethicones and polydimethylsiloxanes modified by aliphatic groups and/or functional groups, such as hydroxyl, thiol, carboxylic acid and/or amine groups.

Mention may be made, as examples of nonvolatile linear hydrocarbon oils, of:
- synthetic esters, such as the oils of formula R1COOR2 wherein R1 represents a residue of a linear or branched fatty acid comprising from 1 to 40 carbon atoms and R2 represents a hydrocarbon chain, in particular a branched hydrocarbon chain containing from 1 to 40 carbon atoms, on condition that the sum of the number of carbon atoms of the R1 and R2 chains is greater than or equal to 10; the esters may be, in particular, chosen from esters of fatty acid and alcohol, such as for example cetostearyl octanoate, esters of isopropyl alcohol, such as isopropyl myristate or isopropyl palmitate, ethyl palmitate, 2-ethylhexyl palmitate, isopropyl stearate or isopropyl isostearate,
   isostearyl isostearate, octyl stearate, hydroxylated esters, for instance isostearyl lactate or octyl hydroxystearate, diisopropyl adipate, heptanoates, and in particular isostearyl heptanoate, alcohol or polyalcohol octanoates, decanoates or ricinoleates, for instance propylene glycol dioctanoate, cetyl octanoate or tridecyl octanoate, 2-ethylhexyl 4-diheptanoate and 2-ethylhexyl palmitate, alkyl benzoate, polyethylene glycol diheptanoate, propylene glycol 2-diethylhexanoate, and mixtures thereof, C₁₂-C₁₅ alcohol benzoates, hexyl laurate, neopentanoic acid esters, for instance isodecyl neopentanoate, isotridecyl neopentanoate, isostearyl neopentanoate, octyldodecyl neopentanoate, isononanoic acid esters, for instance isononyl isononanoate, isotridecyl isononanoate, octyl isononanoate, hydroxylated esters, for instance isostearyl lactate and diisostearyl malate; and more particularly isodecyl neopentanoate;
- dialkyl carbonates, the two alkyl chains possibly being identical or different, such as dicaprylyl carbonate sold under the name Cetiol CC^{®} by Cognis;
- synthetic ethers having from 10 to 40 carbon atoms, such as dicaprylyl ether.

According to a preferred form, the oily dispersion of silicone elastomer containing carboxylic acid functions comprises from 10% to 40% by weight of silicone elastomer active material, and more preferentially from 20% to 35% by weight relative to the total weight of the oily dispersion.

According to a preferred form, the composition of the invention comprises at least one silicone elastomer containing carboxylic acid functions in dispersion in isododecane and in gel form. In particular, said silicon elastomer is chosen from compounds 1 and 4 to 11 as defined previously.

According to one particular preferred form, the composition of the invention comprises compound 11 in dispersion in isododecane and in gel form, and more particularly in a content of active material equal to 30% by weight relative to the total weight of the dispersion.

According to another particularly preferred form, the composition of the invention comprises a silicone elastomer containing carboxylic acid functions in dispersion in isododecane and in gel form with the INCI name: ISODODECANE (and) HEXYL/SUCCINYL DIMETHICONE CROSSPOLYMER and more particularly in a content of active material equal to 30% by weight relative to the total weight of the dispersion such as the commercial product DOWSIL EL-7314 SILICONE ELASTOMER BLEND^{®} sold by Dow Corning comprising 30% by weight of active material of said silicone

### Copolymer based on silicone resin and on silicone of dimethiconol type.

The compositions comprise at least one copolymer based on silicone resin and on silicone of dimethiconol type.

Such copolymers are described for example in "Silicone Pressure Sensitive Adhesives", Sobieski and Tangney, Handbook of Pressure Sensitive Adhesive Technology (D. Satas Ed.), Van Nostrand Reinhold, New York.

In the copolymer, the silicone resin is present at a content of between 45% and 75% (relative to the total weight of silicone) and the silicone of dimethiconol type is present at a content of between 25% and 55%, with the sum of the percentages of silicone resin and silicone of dimethiconol type being equal to 100. Preferably, the silicone resin is present at a content of between 55% and 65% (relative to the total weight of silicone) and the silicone of dimethiconol type is present at a content of between 35% and 45%, with the sum of the percentages of silicone resin and silicone of dimethiconol type being equal to 100.

Preferably, the silicone resin according to the invention is the condensation product of SiO₂ groups and R₃(SiO)_{1/2} (triorganosilyl) groups for which each R group is independently selected from methyl, ethyl, propyl or vinyl radicals and for which the ratio between the SiO₂ functions and the R₃(SiO)_{1/2} functions of the silicone resin ranges from 0.6 to 0.9.

Triorganosilyl groups that can be used to form the silicone resin may be trimethylsilyl, triethylsilyl, methylmethylpropylsilyl or dimethylvinylsilyl units and mixtures thereof. The trimethylsilyl group is the preferred group within the context of the invention.

Preferably, the silicone of dimethiconol type according to the invention is a diorganopolysiloxane with terminal OH functions which has a viscosity of between 100 and 100 000 cSt at 25° C and for which the substituents of the diorganopolysiloxane are chosen independently from methyl, ethyl, propyl and vinyl radicals. The diorganopolysiloxanes are preferably linear polymers. Examples of diorganopolysiloxanes may be, without limitation, a polydimethylsiloxane, an ethyl methylpolysiloxane, a copolymer of dimethylsiloxane and methylvinylsiloxane, and mixtures of such polymers or copolymers having OH ends. The preferred diorganopolysiloxane is a polydimethylsiloxane.

Examples of synthesis of such a copolymer are for example described in patent US 5 162 410 or in patent CA 711756.

The copolymers according to the present invention may thus be prepared by heating the following mixture:
- from 45% to 75% by mass of silicone resin which is the condensation product of SiO₂ and R₃(SiO)_{1/2} units for which each R group is independently selected from methyl, ethyl, propyl and vinyl radicals and for which the ratio between the SiO₂ functions and the R₃(SiO)_{1/2} functions of the silicone resin ranges from 0.6 to 0.9;
- from 25% to 55% by mass of diorganopolysiloxane with terminal OH functions which has a viscosity of between 100 and 100 000 cSt at 25° C and for which the substituents of the diorganopolysiloxane are independently chosen from methyl, ethyl, propyl and vinyl radicals;
- from 0.001% to 5% of a suitable catalyst, which is preferably an organic aliphatic amine compound preferably chosen from primary amines, secondary amines, tertiary amines, carboxylic acid salts of the abovementioned amines and quaternary ammonium salts.

The mixture is heated at a temperature of between 80° C and 160° C until the adhesive character of the resultant silicone copolymer is obtained.

The preferred copolymers according to the invention are those with the INCI name TRIMETHYLSILOXYSILICATE/DIMETHICONOL CROSSPOLYMER such as the products sold by Dow Corning under the references DOWSIL FC 5002 ID RESIN GUM^{®}, DOWSIL FC-5001 CM RESIN GUM^{®}, DOWSIL FC 5004 RESIN GUM^{®}, DOWSIL FC 5002 ID RESIN GUM^{®} (Dow Corning Chemical Company); and more particularly DOWSIL FC 5002 ID RESIN GUM^{®}/DOW CORNING.

The composition according to the invention advantageously has a content of copolymer based on silicone resin and silicone of dimethiconol type ranging from 0.2% to 60% by weight relative to the weight of the composition. Preferably, this content ranges from 1% to 30% by weight relative to the weight of the composition.

More specifically, the copolymer(s) based on silicon resin and on silicone of dimethiconol type may notably be present in the composition according to the invention in a content greater than 1.0% and up to 40% by weight relative to the total weight of the composition, preferably ranging from 1.5% to 20% by weight, and preferentially ranging from 1.5% to 15% by weight.

### Monoalcohol

According to a specific form of the invention, the compositions of the invention additionally comprise at least one linear or branched monoalcohol containing from 2 to 6 carbon atoms, and in particular from 2 to 4 carbon atoms.

The compositions of the invention can comprise one or more monoalcohol(s).

This monoalcohol can be represented, for example, by the formula RaOH, in which Ra represents a linear or branched alkyl group comprising from 2 to 6 carbon atoms.

Mention may be made, as monoalcohol, of ethanol, isopropanol, propanol or butanol.

According to one embodiment, the compositions of the invention comprise isopropanol, ethanol, and mixtures thereof.

According to an advantageous embodiment, the amount of monoalcohol(s) varies from 0.1% to 10% by weight, preferably from 1% to 5% by weight relative to the total weight of said composition.

### Pigments

According to a specific form of the invention, the composition additionally comprises at least one pigment.

The term "pigments" means white or coloured, mineral or organic particles, which are insoluble in an aqueous medium, and which are intended to colour and/or opacify the resulting composition and/or deposit. These pigments may be white or coloured, and mineral and/or organic.

Preferably, the composition comprises at least 5% by weight of pigment(s), more preferentially from 5% to 40% by weight of pigment(s), in particular from 10% to 30% by weight and preferably from 10% to 20% by weight of pigment(s), with respect to the total weight of said composition.

According to a specific embodiment, the pigments used according to the invention are chosen from mineral pigments.

The term "mineral pigment" means any pigment that satisfies the definition in Ullmann's encyclopedia in the chapter on inorganic pigments. Among the mineral pigments that are useful in the present invention, mention may be made of zirconium oxide or cerium oxide, and also zinc oxide, iron oxide (black, yellow or red) or chromium oxide, manganese violet, ultramarine blue, chromium hydrate and ferric blue, titanium dioxide, and metal powders, for instance aluminium powder and copper powder. The following mineral pigments may also be used: Ta₂O₅, Ti₃O₅, Ti₂O₃, TiO, ZrO₂ as a mixture with TiO₂, ZrO₂, Nb₂O₅, CeO₂, ZnS.

The size of the pigment that is useful in the context of the present invention is generally greater than 100 nm and may range up to 10 µm, preferably from 200 nm to 5 µm and more preferentially from 300 nm to 1 µm.

According to a particular form of the invention, the pigments have a size characterized by a D[50] greater than 100 nm and possibly ranging up to 10 µm, preferably from 200 nm to 5 µm and more preferentially from 300 nm to 1 µm.

The sizes are measured by static light scattering using a commercial MasterSizer 3000^{®} particle size analyzer from Malvern, which makes it possible to determine the particle size distribution of all of the particles over a wide range which may extend from 0.01 µm to 1000 µm. The data are processed on the basis of the standard Mie scattering theory. This theory is the most suitable for size distributions ranging from submicron to multimicron; it allows an "effective" particle diameter to be determined. This theory is notably described in the publication by Van de Hulst, H.C., Light Scattering by Small Particles, Chapters 9 and 10, Wiley, New York, 1957.

D[50] represents the maximum size that 50% by volume of the particles have.

In the context of the present invention, the mineral pigments are more particularly iron oxide and/or titanium dioxide.

As mineral pigments that may be used in the invention, mention may also be made of nacres.

The term "nacres" should be understood as meaning coloured particles of any form, which may or may not be iridescent, notably produced by certain molluscs in their shell, or alternatively synthesized, and which have a colour effect via optical interference.

The nacres can be chosen from pearlescent pigments, such as titanium oxide-coated mica covered with an iron oxide, titanium oxide-coated mica covered with bismuth oxychloride, titanium oxide-coated mica covered with chromium oxide, titanium oxide-coated mica covered with an organic dye and also pearlescent pigments based on bismuth oxychloride. They may also be mica particles, at the surface of which are superposed at least two successive layers of metal oxides and/or of organic colorants.

Examples of nacres that may also be mentioned include natural mica covered with titanium oxide, with iron oxide, with natural pigment or with bismuth oxychloride.

Among the nacres available on the market, mention may be made of the nacres Timica^{®}, Flamenco^{®} and Duochrome^{®} (based on mica) sold by the company Engelhard, the Timiron^{®} nacres sold by the company Merck, the Prestige^{®} mica-based nacres sold by the company Eckart, and the Sunshine^{®} synthetic mica-based nacres sold by the company Sun Chemical.

The nacres may more particularly have a yellow, pink, red, bronze, orange, brown, gold and/or coppery colour or glint.

As illustrations of nacres that may be used in the context of the present invention, mention may be made notably of the gold-coloured nacres sold notably by the company Engelhard under the name Brilliant Gold 212G^{®} (Timica), Gold 222C^{®} (Cloisonne), Sparkle Gold^{®} (Timica), Gold 4504^{®} (Chromalite) and Monarch Gold 233X^{®} (Cloisonne); the bronze nacres sold notably by the company Merck under the name Bronze Fine^{®} (17384) (Colorona) and Bronze^{®} (17353) (Colorona) and by the company Engelhard under the name Super Bronze (Cloisonne); the orange nacres sold notably by the company Engelhard under the name Orange 363C^{®} (Cloisonne) and Orange MCR 101 (Cosmica) and by the company Merck under the name Passion Orange^{®} (Colorona) and Matte Orange (17449) ^{®} (Microna); the brown-tinted nacres sold notably by the company Engelhard under the name Nu-Antique Copper 340XB^{®} (Cloisonne) and Brown CL4509^{®} (Chromalite); the nacres with a copper glint sold notably by the company Engelhard under the name Copper 340A^{®} (Timica); the nacres with a red glint sold notably by the company Merck under the name Sienna Fine^{®} (17386) (Colorona^{®}); the nacres with a yellow glint sold notably by the company Engelhard under the name Yellow (4502)^{®} (Chromalite); the red-tinted nacres with a gold glint sold notably by the company Engelhard under the name Sunstone G012^{®} (Gemtone); the pink nacres sold notably by the company Engelhard under the name Tan Opale G005^{®} (Gemtone); the black nacres with a gold glint sold notably by the company Engelhard under the name Nu Antique Bronze 240 AB^{®} (Timica), the blue nacres sold notably by the company Merck under the name Matte Blue^{®} (17433) (Microna), the white nacres with a silvery glint sold notably by the company Merck under the name Xirona Silver^{®}, and the golden-green pink-orange nacres sold notably by the company Merck under the name Indian Summer^{®} (Xirona), and mixtures thereof.

Mention may also be made, among the pigments that may be used according to the invention, of those having an optical effect different from a simple conventional colouring effect, that is to say a unified and stabilized effect such as is produced by conventional colorants, such as, for example, monochromatic pigments. For the purposes of the invention, the term "stabilized" means lacking the effect of variability of the colour with the angle of observation or in response to a temperature change.

For example, this material can be chosen from particles with a metallic glint, goniochromatic colouring agents, diffractive pigments, thermochromic agents, optical brighteners, and also fibres, in particular interference fibres. Of course, these various materials can be combined so as to provide the simultaneous display of two effects, indeed even of a novel effect in accordance with the invention.

The particles with a metallic glint which can be used in the invention are in particular chosen from:
- particles of at least one metal and/or of at least one metal derivative,
- particles comprising a monomaterial or multimaterial organic or mineral substrate, at least partially covered with at least one layer with a metallic glint comprising at least one metal and/or at least one metal derivative, and
- the mixtures of said particles.

Among the metals that may be present in said particles, mention may be made, for example, of Ag, Au, Cu, Al, Ni, Sn, Mg, Cr, Mo, Ti, Zr, Pt, Va, Rb, W, Zn, Ge, Te and Se, and mixtures or alloys thereof. Ag, Au, Cu, Al, Zn, Ni, Mo, Cr and their mixtures or alloys (for example, bronzes and brasses) are preferred metals.

The term "metal derivatives" denotes compounds derived from metals, in particular oxides, fluorides, chlorides and sulfides.

Mention may be made, by way of illustration of these particles, of aluminum particles, such as those sold under the names Starbrite 1200 EAC^{®} by Silberline and Metalure^{®} by Eckart.

Mention may also be made of metal powders of copper or of alloy mixtures such as the references 2844 sold by the company Radium Bronze, metallic pigments, for instance aluminium or bronze, such as those sold under the names Rotosafe 700^{®} from the company Eckart, silica-coated aluminium particles sold under the name Visionaire Bright Silver^{®} from the company Eckart, and metal alloy particles, for instance the silica-coated bronze (alloy of copper and zinc) powders sold under the name Visionaire Bright Natural Gold^{®} from the company Eckart.

They may also be particles comprising a glass substrate, such as those sold by the company Nippon Sheet Glass under the names Microglass Metashine^{®}.

The goniochromatic colouring agent may be chosen, for example, from interference multilayer structures and liquid-crystal colouring agents.

Examples of symmetrical interference multilayer structures which can be used in compositions produced in accordance with the invention are, for example, the following structures: Al/SiO₂/Al/SiO₂/Al, pigments having this structure being sold by the company DuPont de Nemours; Cr/MgF₂/Al/MgF₂/Cr, pigments having this structure being sold under the name Chromaflair^{®} by the company Flex; MoS₂/SiO₂/Al/SiO₂/MoS₂; Fe₂O₃/SiO₂/Al/SiO₂/Fe₂O₃, and Fe₂O₃/SiO₂/Fe₂O₃/SiO₂/Fe₂O₃, pigments having these structures being sold under the name Sicopearl^{®} by the company BASF; MoS₂/SiO₂/mica-oxide/SiO₂/MoS₂; Fe₂O₃/SiO₂/mica-oxide/SiO₂/Fe₂O₃; TiO₂/SiO₂/TiO₂ and TiO₂/Al₂O₃/TiO₂; SnO/TiO₂/SiO₂/TiO₂/SnO; Fe₂O₃/SiO₂/Fe₂O₃ SnO/mica/TiO₂/SiO₂/TiO₂/mica/SnO, pigments having these structures being sold under the name Xirona^{®} by the company Merck (Darmstadt). By way of example, these pigments may be the pigments of silica/titanium oxide/tin oxide structure sold under the name Xirona Magic^{®} by the company Merck, the pigments of silica/brown iron oxide structure sold under the name Xirona Indian Summer^{®} by the company Merck and the pigments of silica/titanium oxide/mica/tin oxide structure sold under the name Xirona Caribbean Blue^{®} by the company Merck. Mention may also be made of the Infinite Colors pigments from Shiseido. Different effects are obtained depending on the thickness and the nature of the various layers. Thus, with the Fe₂O₃/SiO₂/Al/SiO₂/Fe₂O₃ structure, the colour changes from green-golden to red-grey for SiO₂ layers of 320 to 350 nm; from red to golden for SiO₂ layers of 380 to 400 nm; from violet to green for SiO₂ layers of 410 to 420 nm; from copper to red for SiO₂ layers of 430 to 440 nm.

Mention may be made, as examples of pigments with a polymeric multilayer structure, of those sold by 3M under the name Color Glitter^{®}.

Use may be made, as liquid crystal goniochromatic particles, for example, of those sold by Chenix and of those sold under the name Helicone^{®} HC by Wacker.

### Hydrophobic coated pigments

According to a specific form of the invention, the compositions according to the invention comprise at least one pigment coated with at least one lipophilic or hydrophobic compound and in particular as described in detail below.

This type of pigment is particularly advantageous in so far as it may be considered in a large amount together with a large amount of water. What is more, in so far as they are treated with a hydrophobic compound, they show a predominant affinity for the oily phase, which can then convey them.

Of course, the compositions according to the invention may at the same time contain uncoated pigments.

The coating may also comprise at least one additional non-lipophilic compound.

For the purposes of the invention, the "coating" of a pigment according to the invention generally denotes the total or partial surface treatment of the pigment with a surface agent, absorbed on, adsorbed on or grafted to said pigment.

The surface-treated pigments can be prepared according to surface treatment techniques of chemical, electronic, mechanochemical or mechanical nature which are well known to those skilled in the art. Commercial products can also be used.

The surface agent can be absorbed on, adsorbed on or grafted to the pigments by solvent evaporation, chemical reaction and creation of a covalent bond.

According to one alternative form, the surface treatment consists of a coating of the pigments.

The coating may represent from 0.1% to 20% by weight and in particular from 0.5% to 5% by weight relative to the total weight of the coated pigment.

The coating may be produced, for example, by adsorption of a liquid surface agent onto the surface of the solid particles by simple mixing with stirring of the particles and of said surface agent, optionally with heating, prior to the incorporation of the particles into the other ingredients of the makeup or care composition.

The coating may be produced, for example, by chemical reaction of a surface agent with the surface of the solid pigment particles and creation of a covalent bond between the surface agent and the particles. This method is notably described in patent US 4 578 266.

The chemical surface treatment may consist in diluting the surface agent in a volatile solvent, dispersing the pigments in this mixture and then slowly evaporating off the volatile solvent, so that the surface agent is deposited on the surface of the pigments.

### Lipophilic or hydrophobic treatment agent

According to a specific embodiment of the invention, the pigments can be coated according to the invention with at least one compound chosen from silicone surface agents; fluorinated surface agents; fluorosilicone surface agents; metal soaps; N-acylamino acids or salts thereof; lecithin and derivatives thereof; isopropyl triisostearyl titanate; isostearyl sebacate; natural plant or animal waxes; polar synthetic waxes; fatty esters; phospholipids; and mixtures thereof.

### Silicone surface agent

According to a specific embodiment, the pigments can be completely or partially surface-treated with a compound of silicone nature.

The silicone surface agents can be chosen from organopolysiloxanes, silane derivatives, silicone-acrylate copolymers, silicone resins and mixtures thereof.

The term "organopolysiloxane compound" is understood to mean a compound having a structure comprising an alternation of silicon atoms and oxygen atoms and comprising organic radicals bonded to the silicon atoms.

### Nonelastomeric organopolysiloxane

Mention may in particular be made, as nonelastomeric organopolysiloxanes, of polydimethylsiloxanes, polymethylhydrosiloxanes and polyalkoxydimethylsiloxanes.

The alkoxy group can be represented by the R-O- radical such that R represents methyl, ethyl, propyl, butyl or octyl, 2-phenylethyl, 2-phenylpropyl or 3,3,3-trifluoropropyl radicals, aryl radicals, such as phenyl, tolyl or xylyl, or substituted aryl radicals, such as phenylethyl.

One method which makes it possible to surface-treat pigments with a polymethylhydrosiloxane consists in dispersing the pigments in an organic solvent and in then adding the silicone compound. On heating the mixture, covalent bonds are created between the silicone compound and the surface of the pigment.

According to a preferred embodiment, the silicone surface agent can be a nonelastomeric organopolysiloxane, in particular chosen from polydimethylsiloxanes.

### Alkylsilanes and alkoxysilanes

Silanes having alkoxy functionality are described in particular by Witucki in A Silane Primer, Chemistry and Applications of Alkoxysilanes, Journal of Coatings Technology, 65, 822, pages 57-60, 1993.

Alkoxysilanes, such as the alkyltriethoxysilanes and the alkyltrimethoxysilanes sold under the references Milquet A-137^{®} (OSI Specialities) and Prosil 9202^{®} (PCR), can be used for coating the pigments.

The use of alkylpolysiloxanes having a reactive end group, such as alkoxy, hydroxyl, halogen, amino or imino, is described in the application JP H07-196946. They are also suitable for treating the pigments.

### Silicone-acrylate polymers

Use may be made of grafted silicone-acrylic polymers having a silicone backbone as described in the patents US 5 725 882, US 5 209 924, US 4 972 037, US 4 981 903, US 4 981 902, US 5 468 477 and in the patents US 5 219 560 and EP 0 388 582.

Other silicone-acrylate polymers can be silicone polymers comprising, in their structure, the unit of formula (II) below: 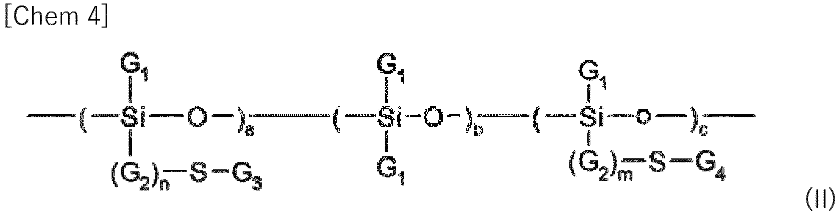 in which the G₁ radicals, which are identical or different, represent hydrogen or a C₁-C₁₀ alkyl radical or else a phenyl radical; the _{G2} radicals, which are identical or different, represent a C₁-C₁₀ alkylene group; G₃ represents a polymeric residue resulting from the (homo)polymerization of at least one ethylenically unsaturated anionic monomer; G4 represents a polymeric residue resulting from the (homo)polymerization of at least one ethylenically unsaturated hydrophobic monomer; m and n are equal to 0 or 1; a is an integer ranging from 0 to 50; b is an integer which may be between 10 and 350 and c is an integer ranging from 0 to 50, with the proviso that one of the parameters a and c is other than 0.

Preferably, the unit of formula (I) above exhibits at least one and more preferentially still all of the following characteristics:
- the G₁ radicals denote an alkyl radical, preferably the methyl radical;
- n is nonzero, and the G₂ radicals represent a divalent C₁-C₃ radical, preferably a propylene radical;
- G₃ represents a polymeric radical resulting from the (homo)polymerization of at least one monomer of the ethylenically unsaturated carboxylic acid type, preferably acrylic acid and/or methacrylic acid;
- G₄ represents a polymeric radical resulting from the (homo)polymerization of at least one monomer of the (C₁-C₁₀)alkyl (meth)acrylate type, preferably of the isobutyl or methyl (meth)acrylate type.

Examples of silicone polymers corresponding to the formula (I) are in particular polydimethylsiloxanes (PDMSs) to which mixed polymer units of the poly(meth)acrylic acid type and of the polymethyl (meth)acrylate type are grafted via a thiopropylene-type connecting link.

Other examples of silicone polymers corresponding to the formula (I) are in particular polydimethylsiloxanes (PDMSs) to which polymer units of the polyisobutyl (meth)acrylate type are grafted via a thiopropylene-type connecting link.

### Silicone resins

The silicone surface agent may be chosen from silicone resins.

The term "resin" is understood to mean a three-dimensional structure.

The silicone resins can be soluble or swellable in silicone oils. These resins are crosslinked polyorganosiloxane polymers.

The nomenclature of silicone resins is known under the name "MDTQ", the resin being described as a function of the various siloxane monomer units which it comprises, each of the letters "MDTQ" characterizing one type of unit.

The letter M represents the monofunctional unit of formula (CH₃)₃SiO_{1/2}, the silicon atom being connected to only one oxygen atom in the polymer comprising this unit.

The letter D means a difunctional unit (CH₃)₂SiO_{2/2} in which the silicon atom is connected to two oxygen atoms.

The letter T represents a trifunctional unit of formula

(CH₃)SiO_{3/2}.

In the units M, D and T defined above, at least one of the methyl groups can be replaced by an R group other than the methyl group, such as a hydrocarbon (in particular alkyl) radical having from 2 to 10 carbon atoms or a phenyl group or alternatively a hydroxyl group.

Finally, the letter Q means a tetrafunctional unit SiO_{4/2} in which the silicon atom is bonded to four oxygen atoms, themselves bonded to the remainder of the polymer.

Various resins with different properties can be obtained from these different units, the properties of these polymers varying as a function of the type of monomers (or units), of the type and number of radicals replaced, of the length of the polymer chain, of the degree of branching and of the size of the pendent chains.

Mention may be made, as examples of these silicone resins, of:
- siloxysilicates, which can be trimethylsiloxysilicates of formula [(CH₃)₃SiO_{1/2}]x(SiO_{4/2})y (MQ units) in which x and y are integers ranging from 50 to 80;
- polysilsesquioxanes of formula (CH₃SiO_{3/2})x (T units) in which x is greater than 100 and at least one of the methyl radicals of which can be replaced by an R group as defined above;
- polymethylsilsesquioxanes, which are polysilsesquioxanes in which none of the methyl radicals is replaced by another group.

Such polymethylsilsesquioxanes are described in the document US 5 246 694.

Mention may be made, as examples of commercially available polymethylsilsesquioxane resins, of those which are sold:
- by the company Wacker under the reference Resin MK^{®}, such as Belsil PMS MK^{®}: polymer comprising CH₃SiO_{3/2} repeating units (T units), which may also comprise up to 1% by weight of (CH₃)₂SiO_{2/2} units (D units) and having an average molecular weight of about 10 000;
- by Shin-Etsu under the reference KR-220L^{®}, which are composed of T units of formula CH₃SiO_{3/2} and have Si-OH (silanol) end groups, under the reference KR-242A^{®}, which comprise 98% of T units and 2% of dimethyl D units and have Si-OH end groups, or else under the reference KR-251^{®}, comprising 88% of T units and 12% of dimethyl D units and having Si-OH end groups.

Mention may be made, as siloxysilicate resins, of trimethylsiloxysilicate (TMS) resins optionally in the form of powders. Such resins are sold under the references SR1000^{®}, E 1 170-002^{®} or SS 4230^{®} by the company General Electric or under the references TMS 803^{®}, Wacker 803^{®} and 804^{®} by the company Wacker Silicone Corporation.

Mention may also be made of trimethylsiloxysilicate resins sold in a solvent such as cyclomethicone, which are sold under the names KF-7312J^{®} by Shin-Etsu and DC 749^{®} and DC 593^{®} by Dow Corning.

Mention may be made, as examples of commercial references of pigments treated with a silicone compound, of:
- red iron oxide/dimethicone sold under the reference SA-C 338075-10^{®} by the company Miyoshi Kasei; and
- a pigment obtained by treatment of DC Red 7 with a silicone compound, sold by Coletica under the reference Gransil GCM^{®} (which is a mixture of D5 and Polysilicone-11).

### Fluorinated surface agent

The pigments can be completely or partially surface-treated with a compound of fluorinated nature.

The fluorinated surface agents can be chosen from perfluoroalkyl phosphates, perfluoropolyethers, polytetrafluoroethylenes (PTFEs), perfluoroalkanes, perfluoroalkyl silazanes, polyhexafluoropropylene oxides or polyorganosiloxanes comprising perfluoroalkyl groups or perfluoropolyethers.

The term "perfluoroalkyl radical" is understood to mean an alkyl radical in which all the hydrogen atoms have been replaced with fluorine atoms.

Perfluoropolyethers are described in particular in the patent application EP 0 486 135 and are sold under the trade name Fomblin^{®} by Montefluos.

Perfluoroalkyl phosphates are described in particular in the application JP H05-86984. The perfluoroalkyl phosphate diethanolamines sold by Asahi Glass under the reference Asahi Guard AG530^{®} can be used.

Mention may be made, among the perfluoroalkanes, of perfluorocycloalkanes, perfluoro(alkylcycloalkanes), perfluoropolycycloalkanes, perfluorinated aromatic hydrocarbons (perfluoroarenes) and organoperfluorinated hydrocarbon compounds comprising at least one heteroatom.

Mention may be made, among the perfluoroalkanes, of the series of the linear alkanes, such as perfluorooctane, perfluorononane or perfluorodecane.

Mention may be made, among the perfluorocycloalkanes and perfluoro(alkylcycloalkanes), of perfluorodecalin, sold under the name Flutec PP5 GMP^{®} by Rhodia, perfluoro(methyldecalin) or perfluoro((C₃-C₅)alkylcyclohexanes), such as perfluoro(butylcyclohexane).

Mention may be made, among the perfluoropolycycloalkanes, of bicyclo[3.3.1]nonane derivatives, such as perfluorotrimethylbicyclo[3.3.1]nonane, adamantane derivatives, such as perfluorodimethyladamantane, and perfluorinated derivatives of hydrogenated phenanthrene, such as tetracosafluorotetradecahydrophenanthrene.

Mention may be made, among the perfluoroarenes, of perfluorinated derivatives of naphthalene, such as perfluoronaphthalene and perfluoro-1-methylnaphthalene.

Mention may be made, as examples of commercial references of pigments treated with a fluorinated compound, of:
- yellow iron oxide/perfluoroalkyl phosphate, sold under the reference PF 5 Yellow 601^{®} by Daito Kasei;
- red iron oxide/perfluoroalkyl phosphate, sold under the reference PF 5 Red R 516L^{®} by Daito Kasei;
- black iron oxide/perfluoroalkyl phosphate sold under the reference PF 5 Black BL 100^{®} by the company Daito Kasei;
- titanium dioxide/perfluoroalkyl phosphate sold under the reference PF 5 TiO2 CR 50^{®} by the company Daito Kasei;
- yellow iron oxide/perfluoropolymethyl isopropyl ether sold under the reference Iron Oxide Yellow BF-25-3^{®} by the company Toshiki;
- DC Red 7/perfluoropolymethyl isopropyl ether sold under the reference D&C Red 7 FHC^{®} by the company Cardre Inc.; and
- DC Red 6/PTFE sold under the reference T 9506^{®} by the company Warner-Jenkinson.

### Fluorosilicone surface agent

The pigments can be completely or partially surface-treated with a compound of fluorosilicone nature.

The fluorosilicone compound can be chosen from perfluoroalkyl dimethicones, perfluoroalkylsilanes and perfluoroalkyltrialkoxysilanes.

Mention may be made, as perfluoroalkylsilanes, of the products LP-IT^{®} and LP-4T^{®}, sold by Shin-Etsu Silicone.

The perfluoroalkyl dimethicones can be represented by the following formula: 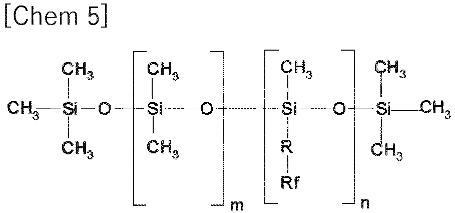 in which:
- R represents a linear or branched divalent alkyl group having from 1 to 6 carbon atoms, preferably a divalent methyl, ethyl, propyl or butyl group;
- Rf represents a perfluoroalkyl radical having from 1 to 9 carbon atoms and preferably from 1 to 4 carbon atoms;
- m is chosen between 0 and 150 and preferably between 20 and 100; and
- n is chosen between 1 and 300 and preferably between 1 and 100.

Mention may be made, as examples of commercial references of pigments treated with a fluorosilicone compound, of titanium dioxide/fluorosilicone, sold under the reference Fluorosil Titanium Dioxide 100TA^{®} by Advanced Dermaceuticals International Inc.

Mention may more particularly be made, as examples of commercial references of pigments treated with a perfluoroalkyltrialkoxysilane compound, for instance perfluorohexylethyltriethoxysilane and perfluorooctyltriethoxysilane, of the following coated iron oxide pigments and coated titanium dioxide pigments:
- Titanium Dioxide (and) Aluminium Hydroxide (and) Perfluorohexylethyl Triethoxysilane/Cl77891 (and) Aluminium Hydroxide (and) Perfluorooctyl Triethoxysilane (FHS-3 TiO₂ CR-50^{®}, Daito Kasei Kogyo);
- Iron oxides (and) Perfluorohexylethyl Triethoxysilane/Cl77492 (and) Perfluorooctyl Triethoxysilane (FHS-5 Sunpuro Yellow C33-9001^{®}, Daito Kasei Kogyo);
- Iron oxides (and) Perfluorohexylethyl triethoxysilane/Cl77491 (and) Perfluorooctyl Triethoxysilane (FHS-5 Sunpuro Red C33-8001^{®}, Daito Kasei Kogyo);
- Iron oxides (and) Perfluorohexylethyl Triethoxysilane/Cl77499 (and) Perfluorooctyl Triethoxysilane (FHS-5 Sunpuro Black C33-7001^{®}, Daito Kasei Kogyo).

### Other lipophilic surface agents

The hydrophobic treatment agent can also be chosen from:
(i) metal soaps, such as aluminium dimyristate and the aluminium salt of hydrogenated tallow glutamate.

Mention may in particular be made, as metal soaps, of metal soaps of fatty acids having from 12 to 22 carbon atoms and in particular those having from 12 to 18 carbon atoms.

The metal of the metal soap can in particular be zinc or magnesium.

Use may be made, as metal soap, of zinc laurate, magnesium stearate, magnesium myristate, zinc stearate and mixtures thereof.

The hydrophobic treatment agent can also be chosen from ii) fatty acids, such as lauric acid, myristic acid, stearic acid or palmitic acid.

The hydrophobic treatment agent can also be chosen from iii) N-acylated amino acids or salts thereof, which can comprise an acyl group having from 8 to 22 carbon atoms, such as, for example, a 2-ethylhexanoyl, caproyl, lauroyl, myristoyl, palmitoyl, stearoyl or cocoyl group.

The amino acid can, for example, be lysine, glutamic acid or alanine.

The salts of these compounds may be the aluminium, magnesium, calcium, zirconium, zinc, sodium or potassium salts.

Thus, according to a particularly preferred embodiment, an N-acylamino acid derivative may in particular be a glutamic acid derivative and/or a salt thereof, and more particularly a stearoyl glutamate, for instance aluminium stearoyl glutamate.

The hydrophobic treatment agent can also be chosen from iv) lecithin and derivatives thereof.

The hydrophobic treatment agent can also be v) isopropyl triisostearyl titanate.

As examples of isopropyl titanium triisostearate (ITT)-treated pigments, mention may be made of those sold under the commercial references BWBO-I2^{®} (Iron oxide Cl77499 and isopropyl titanium triisostearate), BWYO-I2^{®} (Iron oxide CI77492 and isopropyl titanium triisostearate) and BWRO-I2^{®} (Iron oxide CI77491 and isopropyl titanium triisostearate) by the company Kobo.

The hydrophobic treatment agent can also be vi) isostearyl sebacate.

The hydrophobic treatment agent can also be chosen from viii) fatty esters, in particular jojoba esters.

The hydrophobic treatment agent can also be chosen from ix) phospholipids.

The waxes mentioned in the compounds cited above can be those generally used in the cosmetics field, as are defined subsequently.

They can in particular be hydrocarbon, silicone and/or fluorinated, optionally comprising ester or hydroxyl functional groups. They can also be of natural or synthetic origin.

The term "polar wax" is understood to mean a wax containing chemical compounds comprising at least one polar group. Polar groups are well known to a person skilled in the art; they can, for example, be alcohol, ester or carboxylic acid groups. Polyethylene waxes, paraffin waxes, microcrystalline waxes, ozokerite or Fischer-Tropsch waxes are not included among polar waxes.

In particular, polar waxes have a mean Hansen solubility parameter *δ* a at 25° C such that *δ* a > 0 (J/cm³)^{1/2} and better still *δ* a > 1 (J/cm³)^{1/2}: ${δ}_{a} = \sqrt{{δ}_{p}^{2} + {δ}_{h}^{2}}$ where *δ* p and *δ* h are, respectively, the polar contributions and the contributions of types of specific interactions to the Hansen solubility parameters.

The definition of solvents in the three-dimensional solubility space according to Hansen is described in the paper by C.M. Hansen, The three-dimensional solubility parameters, J. Paint Technol., 39, 105 (1967):
- *δ* h characterizes the forces of specific interactions (hydrogen bonding, acid/base or donor/acceptor, etc.);
- *δ* p characterizes the Debye interaction forces between permanent dipoles and also the Keesom interaction forces between induced dipoles and permanent dipoles.

The parameters *δ* p and *δ* h are expressed in (J/cm³)^{1/2}.

A polar wax is notably constituted of molecules comprising, besides carbon and hydrogen atoms in their chemical structure, heteroatoms (such as O, N and P).

Mention may notably be made, as nonlimiting illustration of these polar waxes, of natural polar waxes, such as beeswax, lanolin wax, orange wax, lemon wax and Chinese insect waxes, rice bran wax, carnauba wax, candelilla wax, ouricury wax, cork fibre wax, sugar cane wax, Japan wax, sumac wax or montan wax.

According to a specific embodiment, the pigments can be coated with at least one compound chosen from silicone surface agents; fluorinated surface agents; N-acylated amino acids or salts thereof; isopropyl triisostearyl titanate; perfluoroalkyl trialkoxysilanes; natural plant or animal waxes; fatty esters; and mixtures thereof.

According to a specific embodiment, the pigments can be coated with an N-acylated amino acid and/or a salt thereof, in particular with a glutamic acid derivative and/or a salt thereof, notably a stearoyl glutamate, such as, for example, aluminium stearoyl glutamate. Mention may more particularly be made, as examples of coated pigments according to the invention, of titanium dioxides and iron oxide coated with aluminium stearoyl glutamate, for example sold under the reference NAI^{®} by Miyoshi Kasei.

According to one particularly preferred embodiment, the pigments are coated with a perfluoroalkyl trialkoxysilane such as perfluorohexylethyl triethoxysilane and perfluorooctyl triethoxysilane. Mention may more particularly be made, as examples of coated pigments according to the invention, of titanium dioxides and iron oxide coated with perfluorohexylethyl triethoxysilane or perfluorooctyl triethoxysilane, for example sold under the reference FHS-5 Sunpuro^{®} by the company Daito Kasei Kogyo.

### Pigments not coated with a hydrophobic compound

As stated above, a composition can additionally contain pigments not coated with a lipophilic or hydrophobic compound.

These other pigments can be coated with a hydrophilic compound or be uncoated.

These pigments can be mineral pigments, in particular as defined above.

These pigments can also be organic pigments.

The term "organic pigment" refers to any pigment that satisfies the definition in Ullmann's encyclopedia in the chapter on organic pigments. The organic pigment may notably be chosen from nitroso, nitro, azo, xanthene, quinoline, anthraquinone, phthalocyanine, metal complex type, isoindolinone, isoindoline, quinacridone, perinone, perylene, diketopyrrolopyrrole, thioindigo, dioxazine, triphenylmethane and quinophthalone compounds.

The organic pigment(s) may be chosen, for example, from carmine, carbon black, aniline black, melanin, azo yellow, quinacridone, phthalocyanine blue, sorghum red, the blue pigments codified in the Color Index under the references Cl 42090, 69800, 69825, 73000, 74100 and 74160, the yellow pigments codified in the Color Index under the references Cl 11680, 11710, 15985, 19140, 20040, 21100, 21108, 47000 and 47005, the green pigments codified in the Color Index under the references Cl 61565, 61570 and 74260, the orange pigments codified in the Color Index under the references Cl 11725, 15510, 45370 and 71105, the red pigments codified in the Color Index under the references Cl 12085, 12120, 12370, 12420, 12490, 14700, 15525, 15580, 15620, 15630, 15800, 15850, 15865, 15880, 17200, 26100, 45380, 45410, 58000, 73360, 73915 and 75470, and the pigments obtained by oxidative polymerization of indole or phenol derivatives as described in patent FR 2 679 771.

These pigments can also be in the form of composite pigments, such as are described in the patent EP 1 184 426. These composite pigments may notably be composed of particles including an inorganic core at least partially covered with an organic pigment and at least one binder for fixing the organic pigments to the core.

The pigment may also be a lake. The term "lake" means insolubilized dyes adsorbed onto insoluble particles, the assembly thus obtained remaining insoluble during use.

The inorganic substrates onto which the dyes are adsorbed are, for example, alumina, silica, calcium sodium borosilicate or calcium aluminium borosilicate and aluminium.

Among the organic dyes, mention may be made of cochineal carmine. Mention may also be made of the products known under the following names: D&C Red 21 (Cl 45 380), D&C Orange 5 (Cl 45 370), D&C Red 27 (Cl 45 410), D&C Orange 10 (Cl 45 425), D&C Red 3 (Cl 45 430), D&C Red 4 (Cl 15 510), D&C Red 33 (Cl 17 200), D&C Yellow 5 (Cl 19 140), D&C Yellow 6 (Cl 15 985), D&C Green (Cl 61 570), D&C Yellow 10 (Cl 77 002), D&C Green 3 (Cl 42 053), D&C Blue 1 (Cl 42 090).

Mention may be made, as examples of lakes, of the product known under the name D&C Red 7 (Cl 15 850:1).

### Add itives

The compositions according to the invention can additionally comprise additives commonly used in care and/or makeup products, such as UV sunscreens; moisturizing agents, for instance polyols such as glycerol, propanediol or pentylene glycol; fillers; fat-soluble colorants; thickeners or gelling agents; preservatives; fragrances; and mixtures thereof.

### Fillers

The compositions in accordance with the invention can also comprise at least one filler, of organic or mineral nature, which makes it possible in particular to give them additional properties of improved stability, wear property, coverage and/or mattness.

The term "filler" should be understood as meaning colourless or white solid particles of any shape, which are in a form that is insoluble and dispersed in the medium of the composition. These particles, of mineral or organic nature, give body or rigidity to the composition and/or softness and uniformity to the makeup.

The fillers used in the compositions according to the present invention may be in lamellar, globular or spherical form, in the form of fibres or in any other intermediate form between these defined forms.

The fillers according to the invention may or may not be surface-coated, and in particular they may be surface-treated with silicones, amino acids, fluoro derivatives or any other substance that promotes the dispersion and compatibility of the filler in the composition.

Mention may be made, as examples of inorganic fillers, of talc, mica, silica, hollow silica microspheres, kaolin, calcium carbonate, magnesium carbonate, hydroxyapatite, boron nitride, glass or ceramic microcapsules, composites of silica and of titanium dioxide, such as the TSG^{®} series sold by Nippon Sheet Glass, or hydrophobic silica aerogels.

Examples of organic fillers that may be mentioned include polyamide powders (Nylon^{®} Orgasol from Atochem), polyethylene powders, polymethyl methacrylate powders, polytetrafluoroethylene (Teflon^{®}) powders, acrylic acid copolymer powders (Polytrap^{®} from the company Dow Corning), lauroyl lysine, hollow polymer microspheres such as those of polyvinylidene chloride/acrylonitrile, for instance Expancel^{®} (Nobel Industries), Hexamethylene Diisocyanate/Trimethylol Hexyllactone copolymer powder (Plastic Powder^{®} from Toshiki), silicone resin microbeads (for example Tospearl^{®} from Toshiba), synthetic or natural micronized waxes, metal soaps derived from organic carboxylic acids containing from 8 to 22 carbon atoms and preferably from 12 to 18 carbon atoms, for example zinc stearate, magnesium stearate, lithium stearate, zinc laurate or magnesium myristate, Polypore L 200^{®} (Chemdal Corporation), or crosslinked elastomeric organopolysiloxane powders coated with silicone resin, notably with silsesquioxane resin, as described, for example, in patent US 5 538 793. The filler may also be a cellulose powder, such as that sold by Daito in the Cellulobeads range.

### Additional colorants

A composition according to the invention may also comprise at least one additional colorant, preferably in a proportion of at least 0.01% by weight relative to the total weight of the composition.

For obvious reasons, this amount is liable to vary significantly with regard to the intensity of the desired colour effect and of the colour intensity afforded by the colorants under consideration, and its adjustment clearly falls within the competence of a person skilled in the art.

The additional colorants that are suitable for the invention may be fat-soluble.

For the purposes of the invention, the term "fat-soluble colorant" is intended to mean any natural or synthetic, generally organic compound, which is soluble in an oily phase or in solvents that are miscible with a fatty substance, and which is capable of imparting colour.

Mention may in particular be made, as fat-soluble dyes suitable for the invention, of synthetic or natural fat-soluble dyes, such as, for example, DC Red 17, DC Red 21, DC Red 27, DC Green 6, DC Yellow 11, DC Violet 2, DC Orange 5, Sudan red, carotenes (*β* -carotene, lycopene), xanthophylls (capsanthin, capsorubin, lutein), palm oil, Sudan brown, quinoline yellow, annatto or curcumin.

### Cosmetic compositions

The present invention also relates to a cosmetic composition comprising, in a physiologically acceptable medium, a composition as defined above.

The term "physiologically acceptable medium" is understood to denote a medium which is particularly suitable for the application of a composition of the invention to the skin.

The physiologically acceptable medium is generally adapted to the nature of the support onto which the composition is to be applied, and also to the form in which the composition is to be packaged.

### Applications

According to one embodiment, a composition of the invention can advantageously be provided in the form of a composition for caring for the skin of the body or of the face, in particular of the face.

According to another embodiment, a composition of the invention can advantageously be provided in the form of a composition for making up keratin materials, in particular the skin of the body or of the face, in particular of the face.

Thus, according to a sub-mode of this embodiment, a composition of the invention can advantageously be provided in the form of a base composition for makeup.

A composition of the invention can advantageously be provided in the form of a foundation.

According to another sub-mode of this embodiment, a composition of the invention can advantageously be provided in the form of a composition for making up the skin and in particular the face. It can thus be an eyeshadow or a face powder.

Such compositions are notably prepared according to the general knowledge of a person skilled in the art.

Throughout the description, including the claims, the term "including a" should be understood as being synonymous with "including at least one", unless otherwise specified.

The expressions "between ... and ..." and "ranging from ... to ..." should be understood as meaning limits included, unless otherwise specified.

The invention is illustrated in greater detail by the examples and figures presented below. Unless otherwise indicated, the amounts indicated are expressed as weight percentages.

### Example A (invention) and exemple B (outside the invention)

The following compositions were prepared.

**[Table 1]**

| **Phase** | **Ingredients** | **Formula A (invention)** | **Formula B (outside the invention)** |
|---|---|---|---|
| **A** | ISODODECANE (and) HEXYL/SUCCINYL DIMETHICONE CROSSPOLYMER (DOWSIL EL-7314 SILICONE ELASTOMER BLEND^{®}/DOW CORNING in gel form in dispersion at 30% by weight in isododecane) | 63 (i.e. 18.9% of polymer active material) | 63 (i.e. 18.9% of polymer active material) |
| **B** | TRIMETHYLSILOXYSILICATE/DIMETHI CONOL CROSSPOLYMER diluted to 40% in isododecane (DOWSIL FC 5002 RESIN GUM^{®}/DOW CORNING) | 25 (i.e. 10% of polymer active material) | - |
| | Isopropyl alcohol | qs | qs |
| **C** | Isododecane | 0 | 24 |
| | Titanium Dioxide (and) Aluminium Hydroxide (and) Perfluorohexylethyl Triethoxisilane/Cl77891 (and) Aluminium Hydroxide (and) Perfluorooctyl Triethoxysilane (FHS-3 TiO2 CR-50^{®}, Daito Kasei Kogyo) | 7.5 | 7.5 |
| | Iron Oxides (and) Perfluorohexylethyl Triethoxysilane/Cl77492 (and) Perfluorooctyl Triethoxysilane (FHS-5 Sunpuro Yellow C33-9001^{®}, Daito Kasei Kogyo) | 1.8 | 1.8 |
| | Iron Oxides (and) Perfluorohexylethyl Triethoxysilane/Cl77491 (and) Perfluorooctyl Triethoxysilane (FHS-5 Sunpuro Red C33-8001^{®}, Daito Kasei Kogyo) | 0.5 | 0.5 |
| | Iron Oxides (and) Perfluorohexylethyl Triethoxysilane/Cl77499 (and) Perfluorooctyl Triethoxysilane (FHS-5 Sunpuro Black C33-7001^{®}, Daito Kasei Kogyo) | 0.2 | 0.2 |

### Procedure

The constituents of phase A were weighed out in the main beaker and placed in a VMI Turbotest EVO^{®} homogenizer with a deflocculating impeller for 15 min at 1500 rpm. The constituents of phase B were weighed out and mixed until completely solubilized. The constituents of phase C were weighed out and mixed until completely dispersed. Phases B then C were subsequently introduced into phase A. The mixture was left stirring for 15 min at 1500 rpm.

The evaluations performed on examples A and B were carried out at least 24 h after the manufacture of the formulae.

### Example 1: Tribology

### Sample preparation

Deposits with a thickness of 50 µm were produced using a spreader on a support constituted of a Lycra^{®} textile bonded to a 25 µm breathable polyurethane film and coated with a biocompatible silicone gel of 11 Shore (SkinAbs, Prevent Transformation^{®}). The deposits were stored at 25° C for 24 h for complete drying.

Spheres of the dry deposits with a 40 mm radius were cut out in order to carry out the tribometer measurement.

### Friction and wear dimensions

The objective of this in vitro dynamic friction test is to quantify the wear property of foundations.

To do so, a deposit of foundation produced on a normalized "Skinabs^{®}" support and which was dry or impregnated with an attacking factor, was subjected to a friction test. The nature of the "Skinabs^{®}" will be described below.

The apparatus used was the CSM^{®} pin-on-disc tribometer which is composed of 2 parts:
- the static upper part on which the normalized "Skinabs^{®}" support was placed,
- the lower part that performs circular movements, onto which the normalized "Skinabs^{®}" containing the deposit of foundation, dry or impregnated with attacking factor, is attached. The tribometer makes it possible to apply a force normal to the plane and a speed of movement.

The friction generated during the rotational movement may give rise to wear phenomena depending on the resistance of the deposit. These result in a greater or lesser loss of material on the "Skinabs^{®}" of the lower part.

The wear property is quantified by means of observation of the loss of material on the "Skinabs^{®}" made-up with the foundation before and after friction.

The deposits are prepared on a "Skinabs^{®}" which is a Lycra^{®} textile bonded to a 25 µm breathable polyurethane film and coated with a biocompatible silicone gel of 11 Shore 00 which facilitates adhesion to the metal parts of the tribometer.

This "bioskin" has 2 advantages:
- it enables a standardized application using a spreader. The homogeneity of the deposits obtained is perfectly satisfactory in the case of foundations;
- a surface tension of 43 mN.m⁻¹, similar to that of skin, evaluated at 54 mN.m⁻¹.

The foundation was spread using a mechanical spreader with a standardized thickness of 50 µm by carrying out 1 pass. After drying for 24 h in the open air, upon judging that the deposit is completely dry, discs of made-up Skinabs^{®} with a diameter of 5 cm were cut using a punch, while being attentive to the damage caused by the cutting of certain formulae. This piece is then bonded to the lower part of the tribometer.

In the same way, the piece of Skinabs^{®} on the upper part of the tribometer was cut as a 1.8 cm diameter disc which was bonded onto a metal support of the same diameter, of powder-compacting cup type.

The friction radius was set at 8 mm, the rotational speed at 36 rpm which is equivalent to a linear speed of 3 cm/s. The stop criterion is 1 turn, repeatable 5 times. The imposed normal load of 1N made it possible to be discriminating in the domain of the foundations tested while having a low contact pressure representative of in vivo.

The acquisition of the images is carried out by means of a photo after each friction turn and observation is carried out by eye, by comparing the condition of the deposit before and after friction.

The deposit was evaluated after each pass, the degree of degradation (detachment, thinning, discolouration) was evaluated throughout the analysis.

### Discussion and Results

The behaviour of the formulae at different concentrations of DOWSIL FC 5002 IDD RESIN GUM^{®}, Dow Corning (0%, 10% active material) were analysed.

**[Table 2]**

| **Composition** | **Formula A (invention)** | **Formula B (outside the invention)** |
|---|---|---|
| **Appearance of the deposit after the 5^{th} turn°** | No wear | Wear down to the support |

Composition A according to the invention was more resistant to friction than composition B outside the invention that did not contain a copolymer based on silicone resin and on silicone of dimethiconol type.

### Example 2: Rheological analyses

The rheological measurements were carried out in an imposed stress rheometer (RheoStress 600^{®}, Haake). The measurements of shear stress and of rate gradient were carried out using a cone sensor (C35/2° Ti L; 222-1871; 35 mm diameter, 2° angle), with a 0.105 mm gap and a measuring plate (MPC 35; 222-1549; 35 mm diameter, sand blasted). The temperature of the gap is controlled using an Eheim Pro 3600/2075020^{®} hydraulic circulation pump and a Haake Universal Temperature Controler System^{®} (UTC) 6001 temperature control system.

An anti-evaporation bell jar was positioned above the plane of the cone and of the plate during the measurement.

The temperature of the gap was set at 25 ± 0.5° C. An isotherm of 120 seconds at this temperature was applied before each measurement. An upward oscillation curve with an amplitude sweep was imposed with a stress ramp ranging from 10⁻³ to 10³ Pa, with a frequency set at 1 Hz with 50 steps.

### Discussion and Results

The rheological behaviour of the formulae A and B was analysed. Measurements of storage modulus G' and loss or dissipation modulus G" made it possible to determine the complex modulus G* and also the phase shift *δ* between the stress and the strain. These data are analysed as a function of the shear stress.

The behaviour of the rheological curves shows that they are materials which only flow if a high enough stress is applied to them.

The value of the complex modulus G* at the plateau preceding the break in the curve, give an idea of the consistency of the product at rest.

The rheological data of the 3 formulae are described in the table below.

**[Table 3]**

| **Test** | **Formula A (invention)** | **Formula B (outside the invention)** |
|---|---|---|
| **Imposed stress for *δ* = 45°** | 350 Pa | 700 Pa |
| **G* at the plateau** | 660 Pa | 2050 Pa |

A lower complex modulus G* over the whole of the measurement range indicates a formula that is more fluid, more supple and more homogeneous irrespective of the force applied. A change from the elastic state to a viscous state that occurs more rapidly during the application means an easier spreading on the skin.

Composition A according to the invention was more fluid, more supple and more homogeneous than composition B outside the invention that did not contain a copolymer based on silicone resin and on silicone of dimethiconol type.

## Claims

1. Composition for caring for and/or making up keratin materials, in particular the skin, comprising, preferably in a physiologically acceptable medium:
a) at least one silicone elastomer containing carboxylic acid functions; and
b) at least one copolymer based on silicone resin and on silicone of dimethiconol type.

2. Composition according to claim 1, **characterized in that** it is anhydrous.

3. Composition according to claim 1 or 2, comprising a content of active material of silicone elastomer containing carboxylic acid functions ranging from 2 to 25 % by weight, preferably ranging from 5% to 15% by weight relative to the weight of the composition.

4. Composition according to any one of the preceding claims, where the silicone elastomer containing carboxylic acid functions is chosen from those corresponding to formula (I) below: 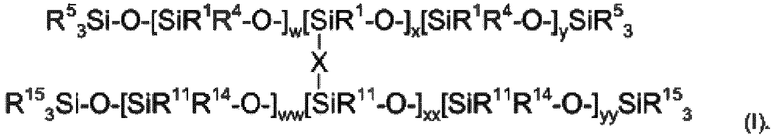 in which
the radical X corresponds to formula (i) below 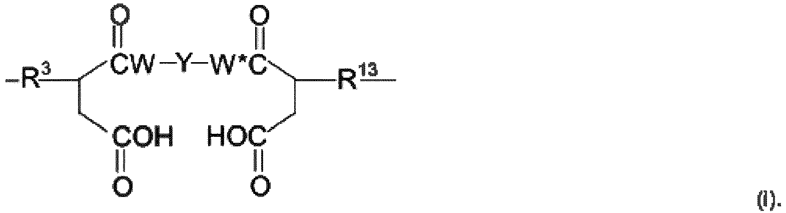 or to the formula (I') below : 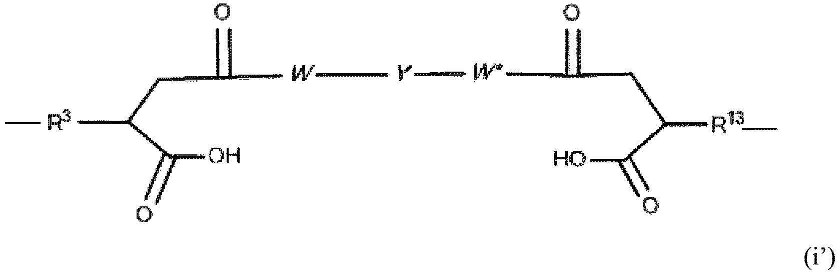 in which
W and W* independently denote an oxygen atom (O) or an N-R group where each
R radical independently denotes a hydrogen atom (H) or an R¹ radical;
Y is a divalent group;
R¹, R¹¹, R⁴, R¹⁴, R⁵ and R¹⁵ independently denote a substituted or unsubstituted hydrocarbyl group;
R³ and R¹³ independently denote a divalent group;
w and ww are, independently, integers ranging from 0 to 1000;
x and xx are, independently, integers ranging from 1 to 100;
y and yy are, independently, integers ranging from 0 to 1000;
preferably, w and y are not simultaneously equal to 0 and ww and yy are not simultaneously equal to 0.

5. Composition according to any one of the preceding claims, where the silicone elastomer of formula (I) is chosen from the compounds 1 to 12 below:

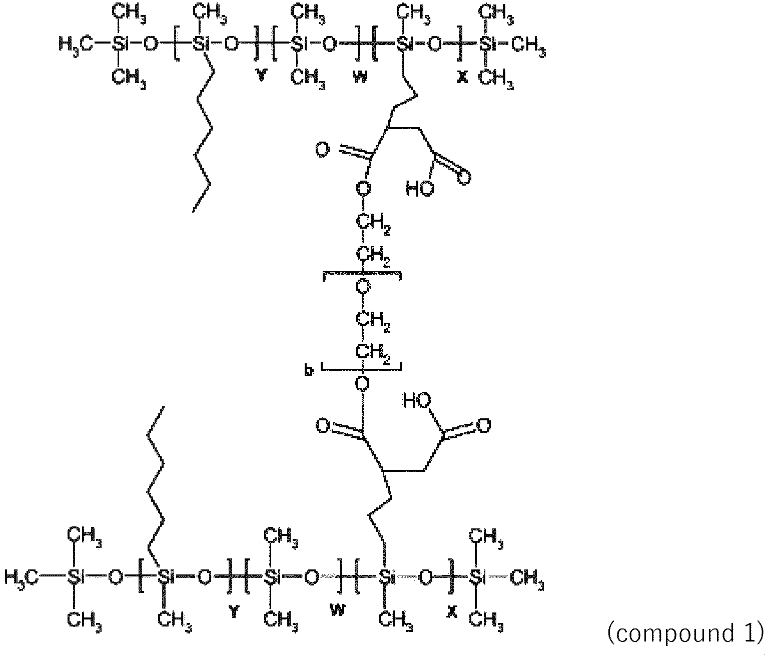

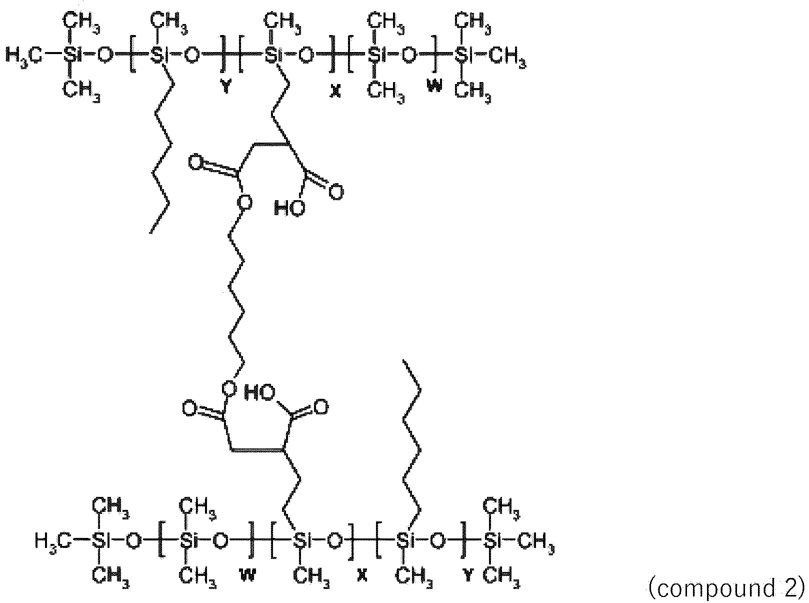

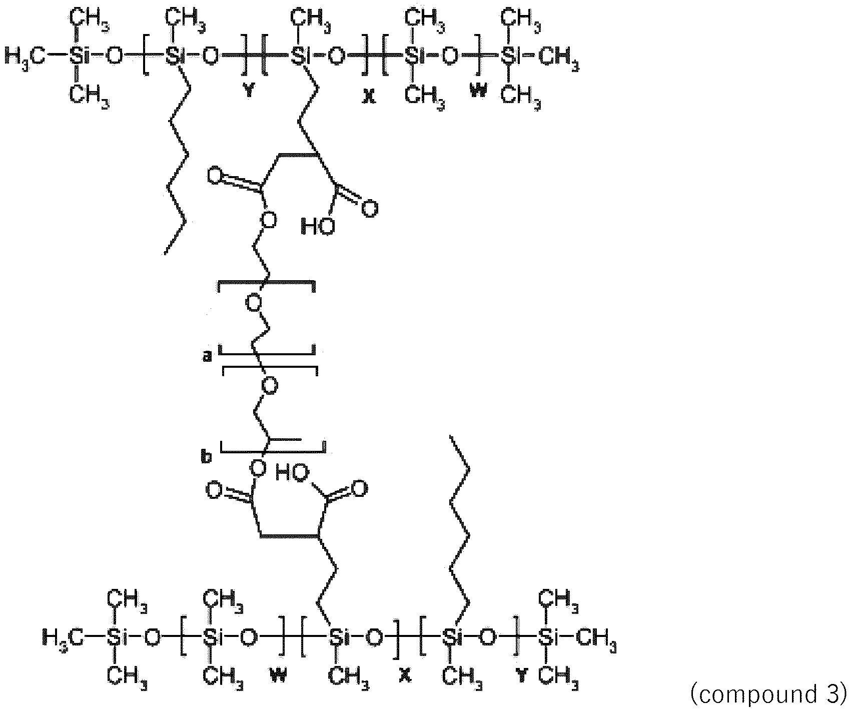

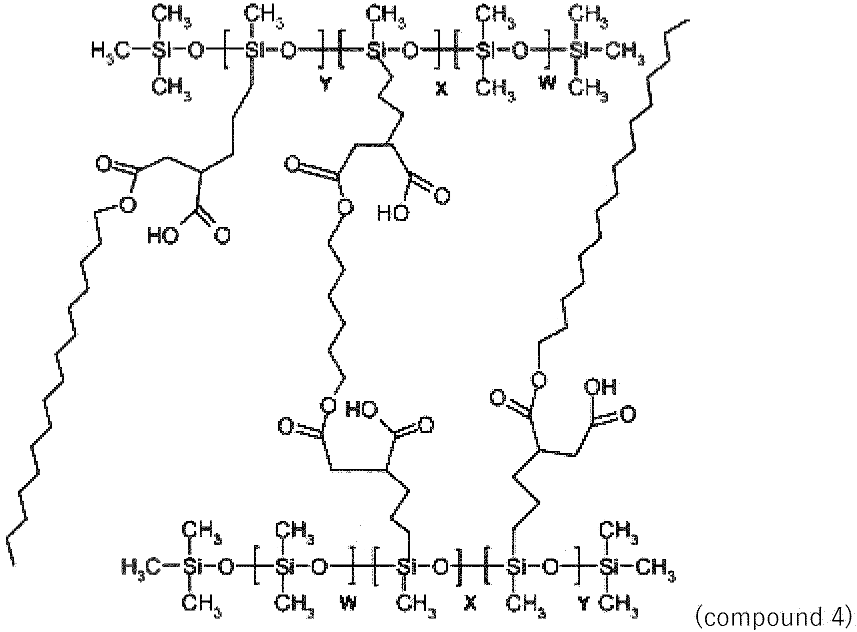

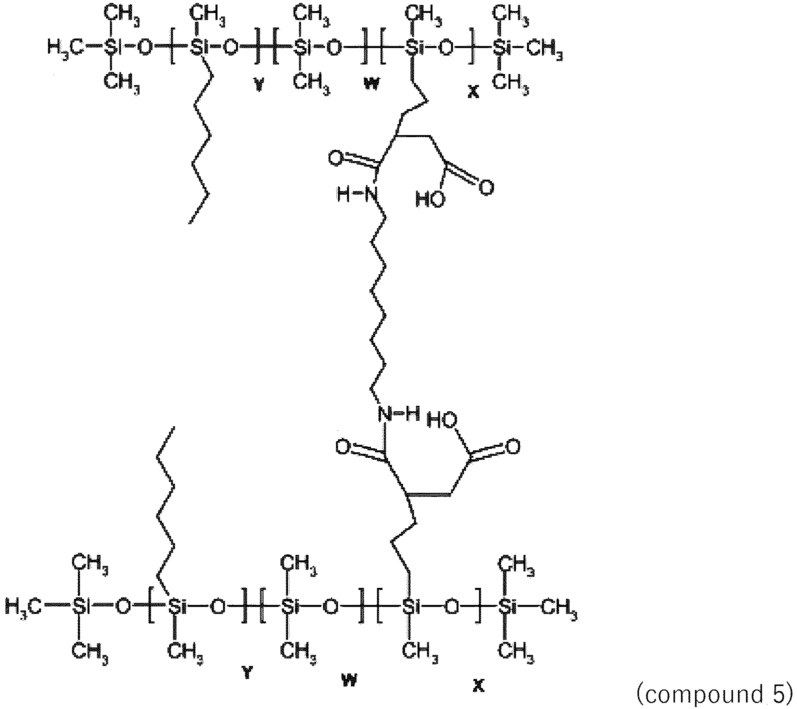

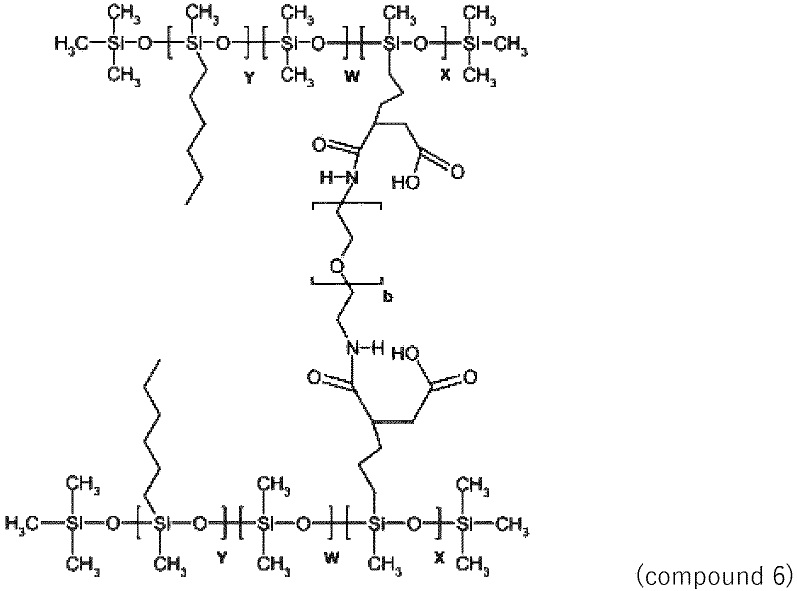

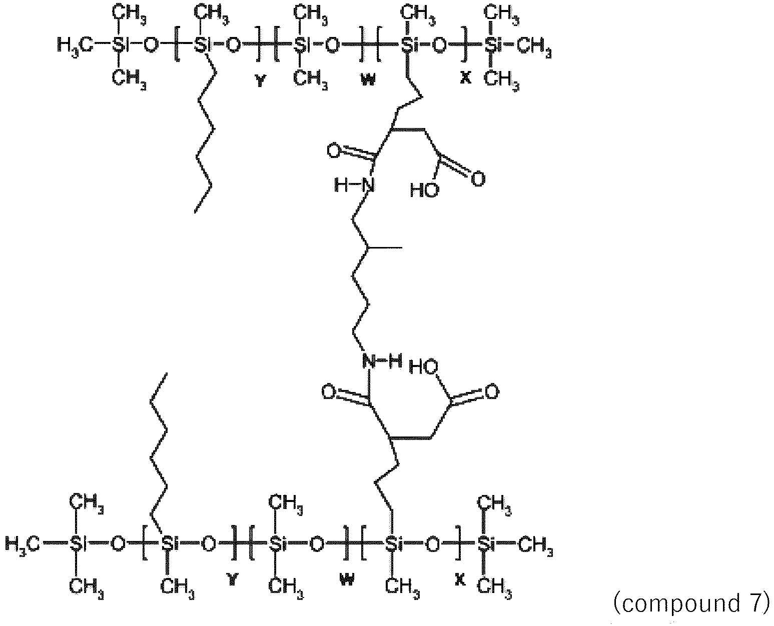

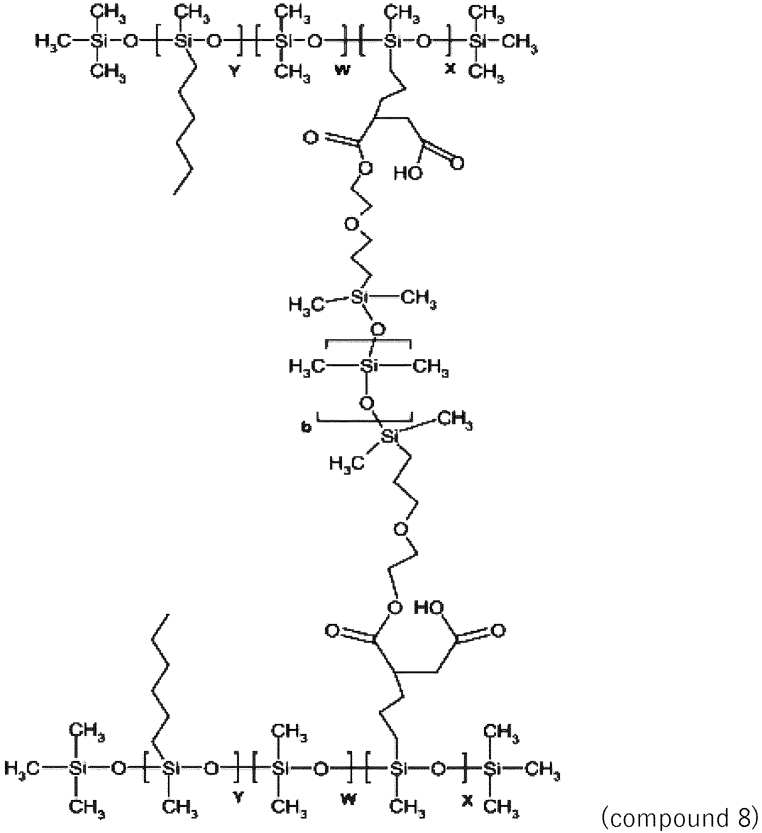

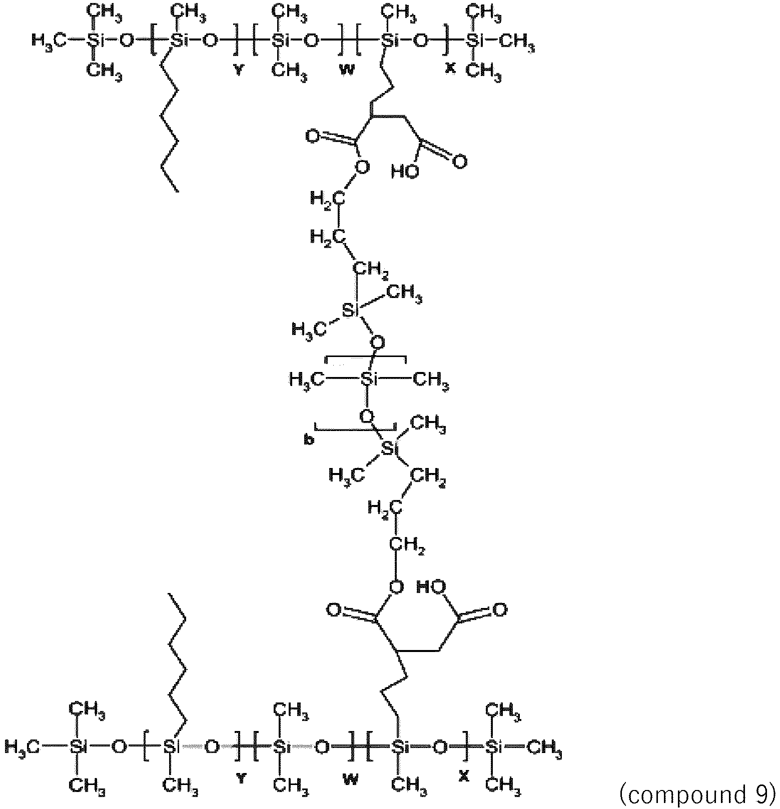

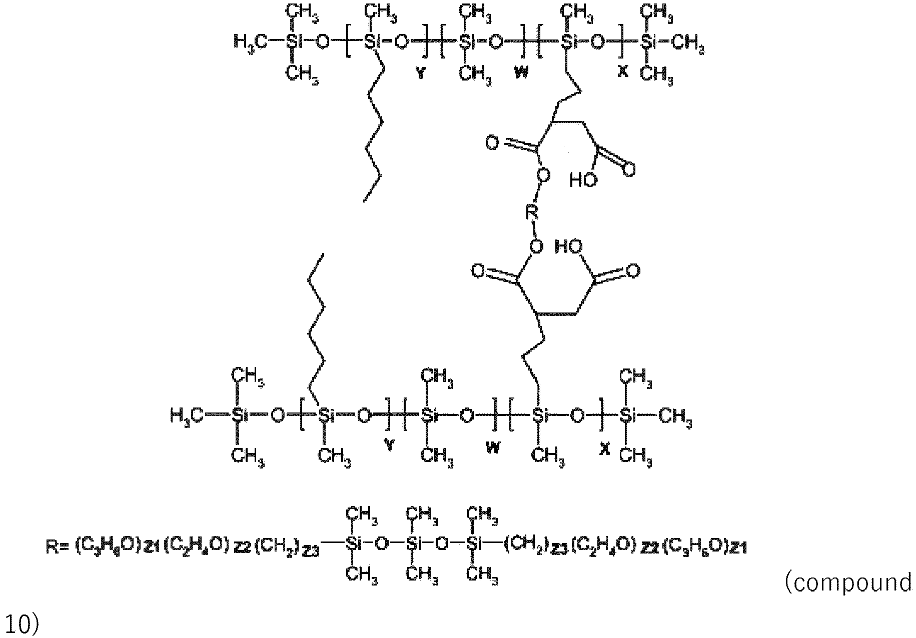

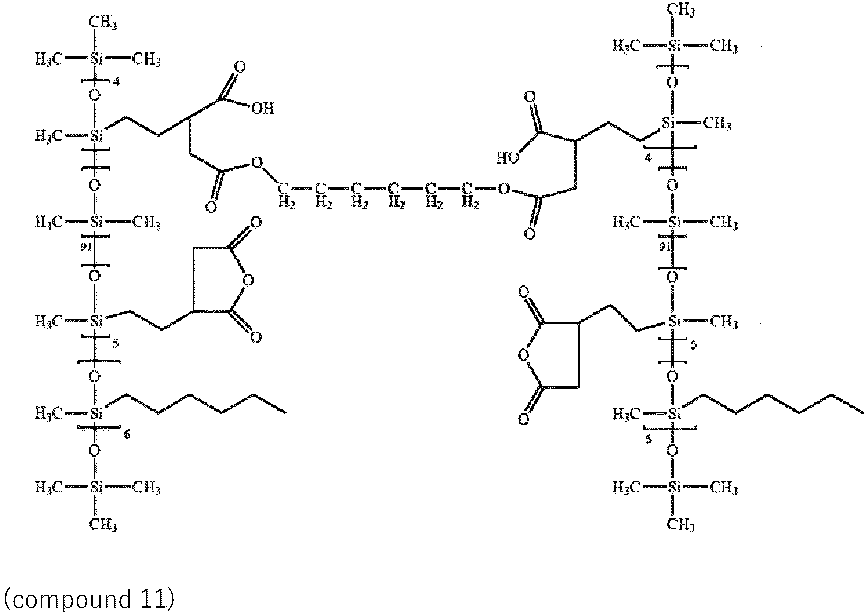

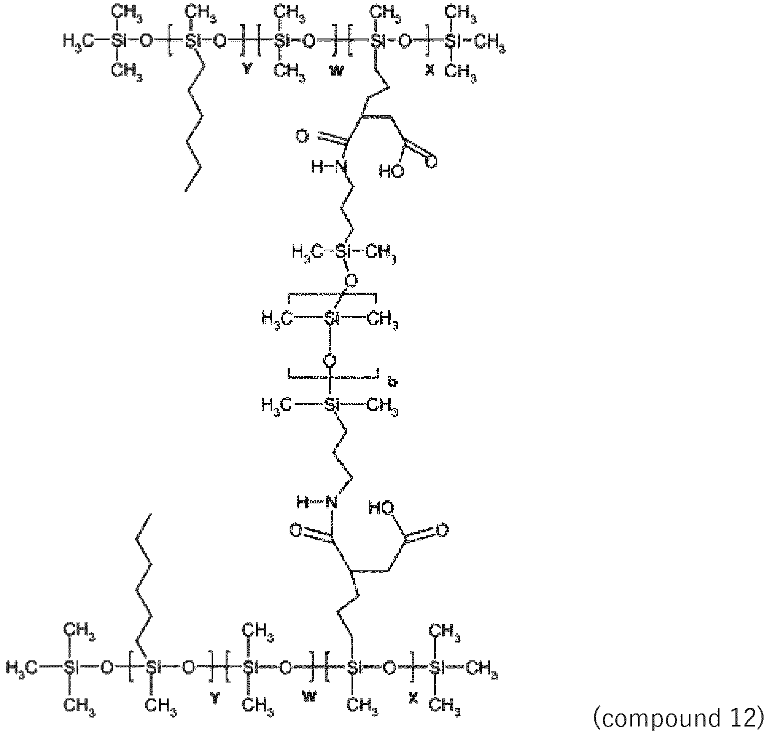

6. Composition according to Claim 5, where the silicone elastomer containing carboxylic acid functions is compound 11.

7. Composition according to any one of Claims 1 to 3, where the silicone elastomer containing carboxylic acid functions is that with the INCI name: HEXYL/SUCCINYL DIMETHICONE CROSSPOLYMER.

8. Composition according to any one of the preceding claims, where the silicone elastomer containing carboxylic acid functions is in dispersion in at least one oil and in gel form; preferably chosen from volatile hydrocarbon oils, volatile silicone oils, non-volatile hydrocarbon oils, non-volatile silicone oils, and mixtures thereof.

9. Composition according to Claim 8, where the oily dispersion of silicone elastomer containing carboxylic acid functions comprises from 10% to 40% by weight of silicone elastomer active material, and more preferentially from 20% to 35% by weight relative to the total weight of the oily dispersion.

10. Composition according to Claim 8 or 9, where the silicone elastomer containing carboxylic acid functions is in dispersion in isododecane and in gel form.

11. Composition according to Claim 10, where the silicone elastomer containing carboxylic acid functions is chosen from compounds 1, 4 and 11, as defined in claim 4, preferably compound 11, and more particularly compound 11 is present in the dispersion based on isododecane having an active material content of 30% by weight.

12. Composition according to Claim 9, comprising at least one silicone elastomer in gel form in dispersion in isododecane having the INCI name: ISODODECANE (and) HEXYL/SUCCINYL DIMETHICONE CROSSPOLYMER, and more particularly having a content of active material of 30% by weight in the isododecane-based dispersion.

13. Composition according to any one of the preceding claims, where the copolymer(s) based on silicone resin and on silicone of dimethiconol type is/are present in a content of greater than 1% and up to 40% by weight relative to the total weight of the composition, preferably ranging from 1.5% to 20% by weight, and more preferentially ranging from 1.5% to 15% by weight.

14. Composition according to any one of the preceding claims, where the copolymer based on silicone resin and on silicone of dimethiconol type is chosen from those with the INCI name: TRIMETHYLSILOXYSILICATE/DIMETHICONOL CROSSPOLYMER.

15. Composition according to any one of the preceding claims, additionally comprising at least one C₂-C₆ monoalcohol, in particular chosen from isopropanol, ethanol, and mixtures thereof.

16. Composition according to Claim 15, where the amount of monoalcohol(s) varies from 0.1% to 10% by weight, preferably from 1% to 5% by weight relative to the total weight of said composition.

17. Composition according to any one of the preceding claims, additionally comprising at least one pigment, preferably chosen from iron oxides, titanium dioxides, and mixtures thereof.

18. Composition according to Claim 17, where the pigment is coated by at least one hydrophobic or lipophilic compound, in particular chosen from silicone surface agents; fluorinated surface agents; fluorosilicone surface agents; metal soaps; N-acylated amino acids or salts thereof; lecithin and derivatives thereof; isopropyl triisostearyl titanate; isostearyl sebacate; natural plant or animal waxes; polar synthetic waxes; fatty esters; phospholipids; and mixtures thereof, more preferentially chosen from fluorosilicone surface agents, even more particularly chosen from perfluoroalkyl trialkoxysilanes.

19. Composition according to Claim 17 or 18, comprising at least 5% by weight of pigment(s), more preferentially from 5% to 40% by weight of pigment(s), in particular from 10% to 30% by weight, and more particularly from 10% to 20% by weight of pigment(s), relative to the total weight of said composition.

20. Process for coating keratin materials, more particularly for making up and/or caring for keratin materials, such as the skin, **characterized in that** it comprises the application to the keratin materials of a composition as defined in any one of the preceding claims.

## Patentansprüche

1. Zusammensetzung zum Pflegen und/oder Schminken von Keratinmaterialien, insbesondere der Haut, umfassend, vorzugsweise in einem physiologisch unbedenklichen Medium:
a) mindestens ein Silikonelastomer mit Carbonsäurefunktionen; und
b) mindestens ein Copolymer auf Basis von Silikonharz und von Silikon vom Dimethiconol-Typ.

2. Zusammensetzung nach Anspruch 1, **dadurch gekennzeichnet, dass** sie wasserfrei ist.

3. Zusammensetzung nach Anspruch 1 oder 2, umfassend einen Aktivsubstanzgehalt an Silikonelastomer mit Carbonsäurefunktionen im Bereich von 2 bis 25 Gew.-%, vorzugsweise im Bereich von 5 bis 15 Gew.-%, bezogen auf das Gewicht der Zusammensetzung.

4. Zusammensetzung nach einem der vorhergehenden Ansprüche, wobei das Silikonelastomer mit Carbonsäurefunktionen aus denjenigen ausgewählt ist, die der nachstehenden Formel (I) entsprechen: 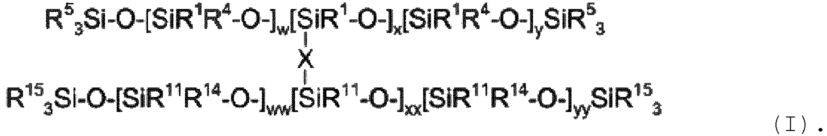 wobei
der Rest X der nachstehenden Formel (i) entspricht: 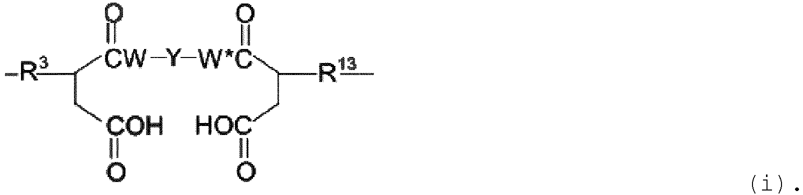
oder der folgenden Formel (I') entspricht: 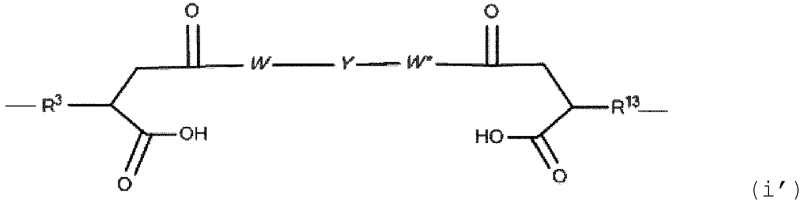
wobei
W und W* unabhängig ein Sauerstoffatom (O) oder eine N-R-Gruppe bedeuten, wobei jeder Rest R unabhängig ein Wasserstoffatom (H) oder einen R¹-Rest bedeutet;
Y eine zweiwertige Gruppe ist;
R¹, R¹¹, R⁴, R¹⁴, R⁵ und R¹⁵ unabhängig voneinander eine substituierte oder unsubstituierte Hydrocarbylgruppe bedeuten;
R³ und R¹³ unabhängig eine zweiwertige Gruppe bedeuten; w und ww unabhängig ganze Zahlen im Bereich von 0 bis 1000 sind;
x und xx unabhängig ganze Zahlen im Bereich von 1 bis 100 sind;
y und yy unabhängig ganze Zahlen im Bereich von 0 bis 1000 sind;
vorzugsweise w und y nicht gleichzeitig gleich 0 sind und ww und yy nicht gleichzeitig gleich 0 sind.

5. Zusammensetzung nach einem der vorhergehenden Ansprüche, wobei das Silikonelastomer der Formel (I) aus den nachstehenden Verbindungen 1 bis 12 ausgewählt ist:

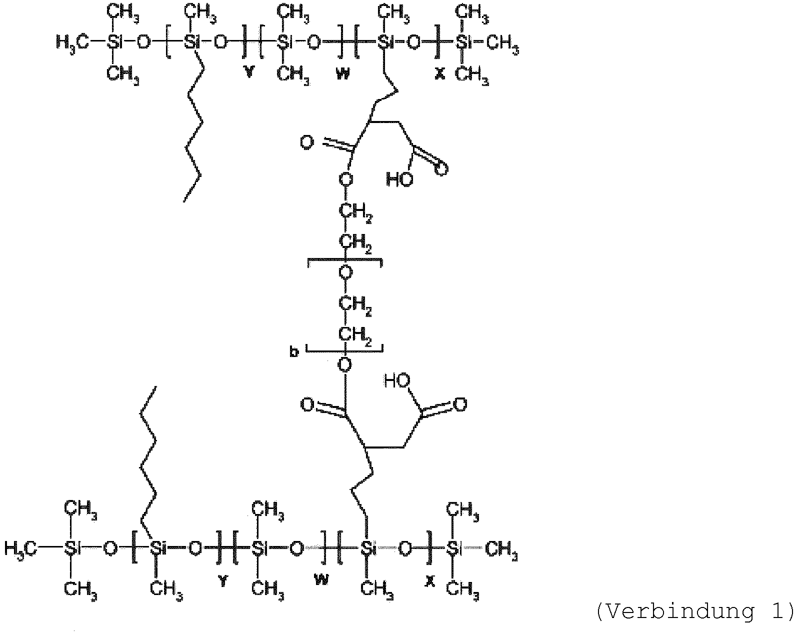

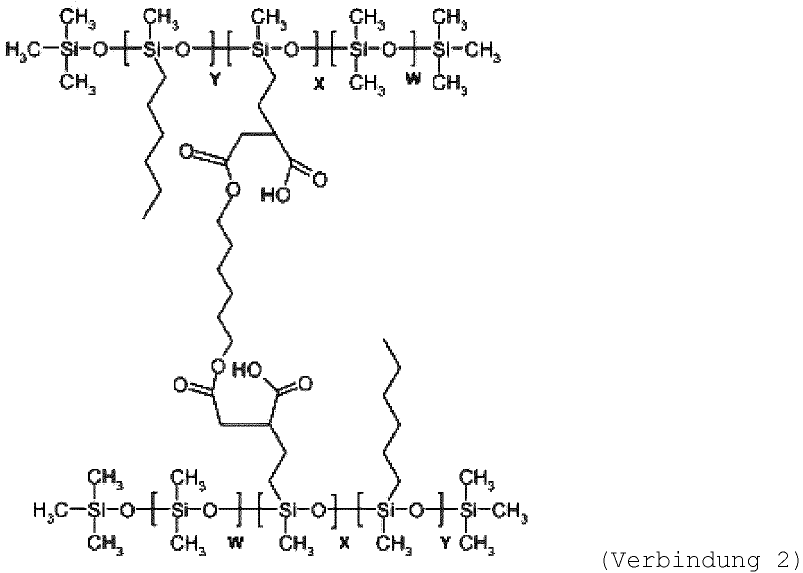

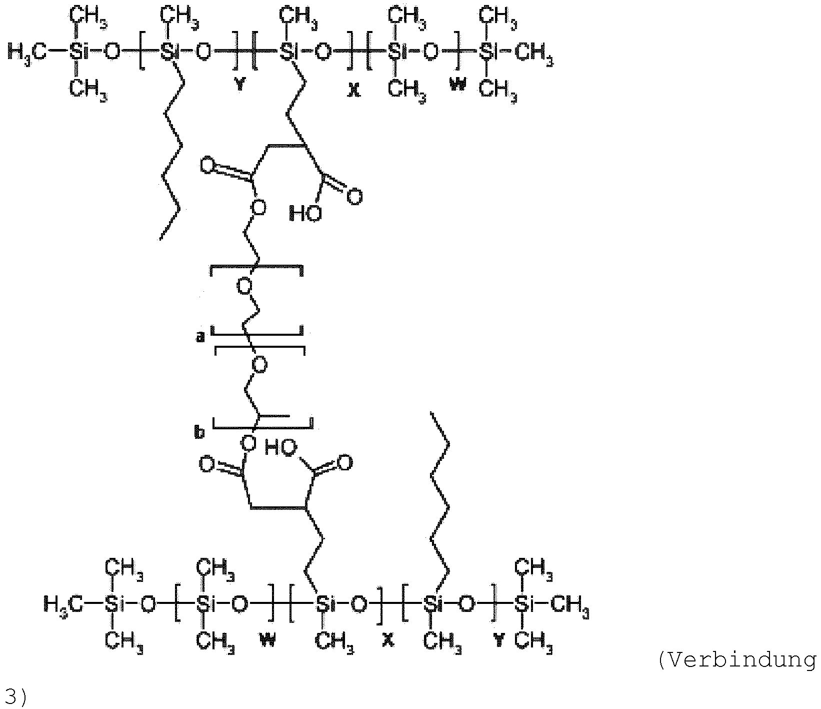

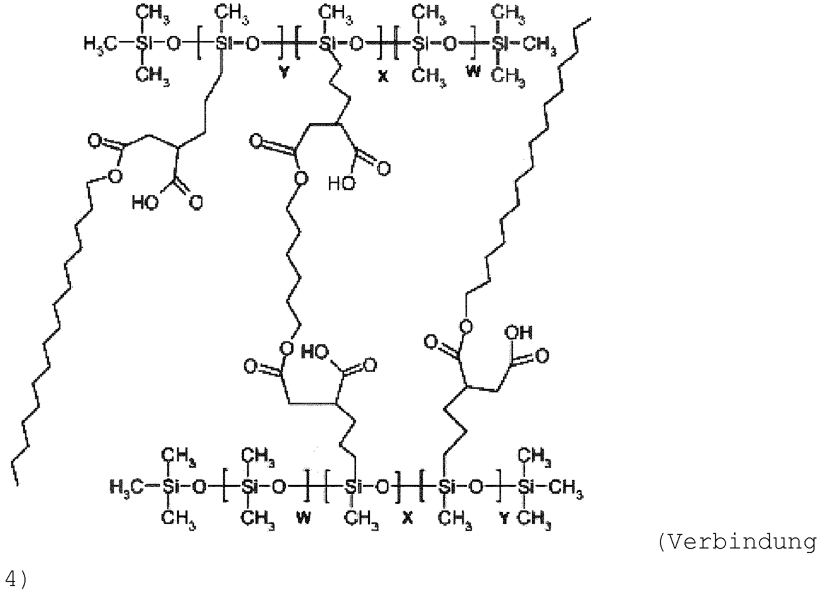

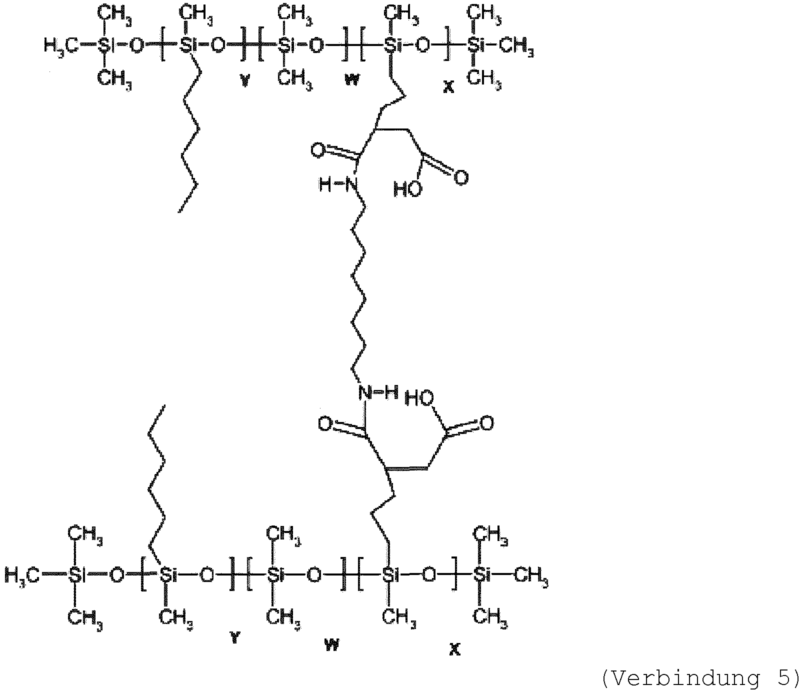

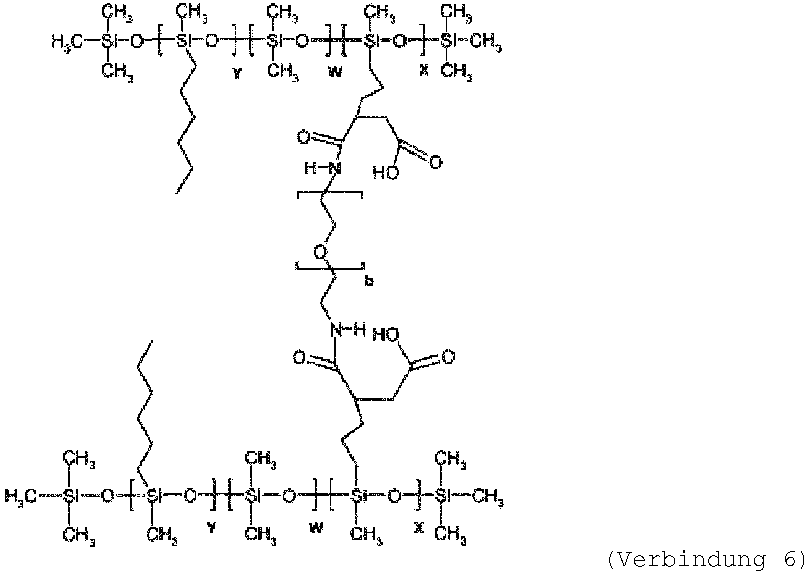

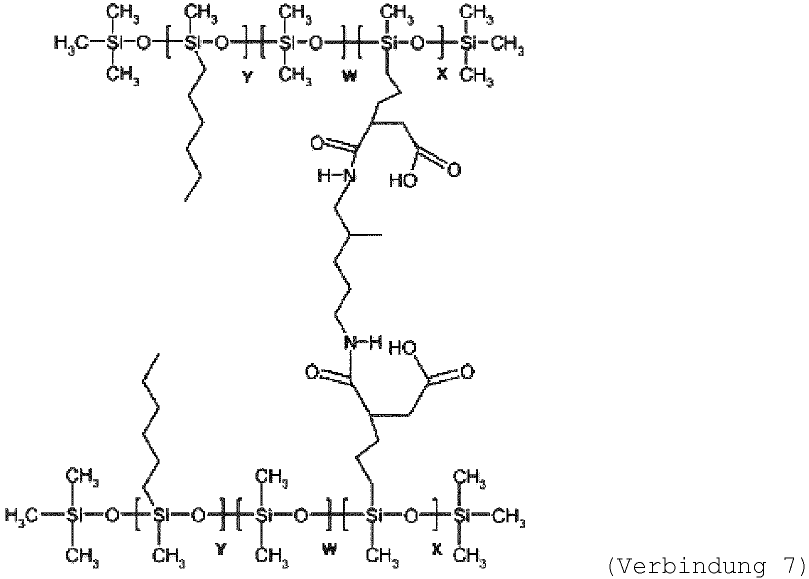

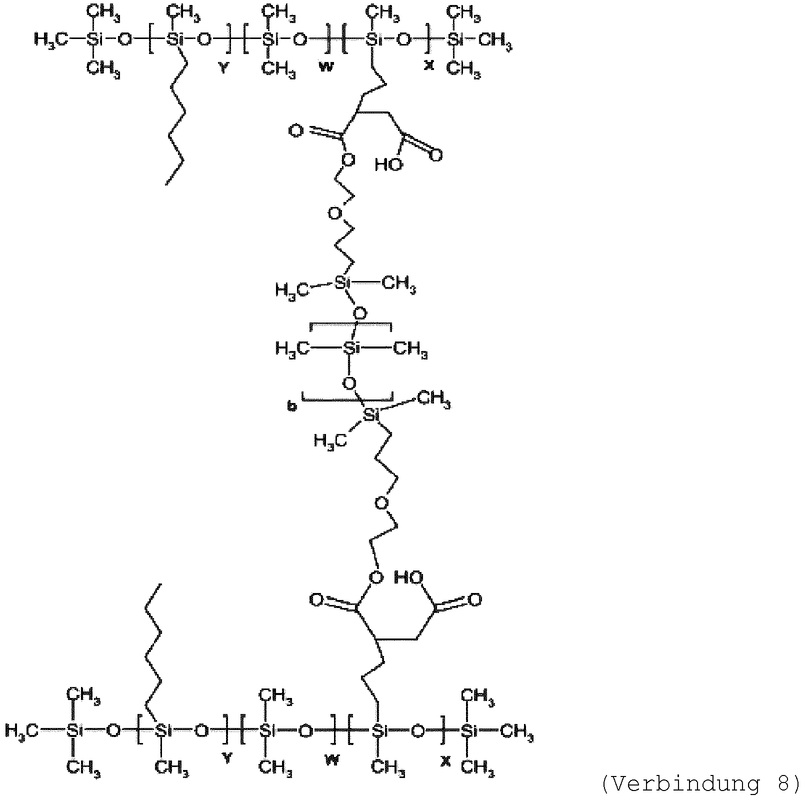

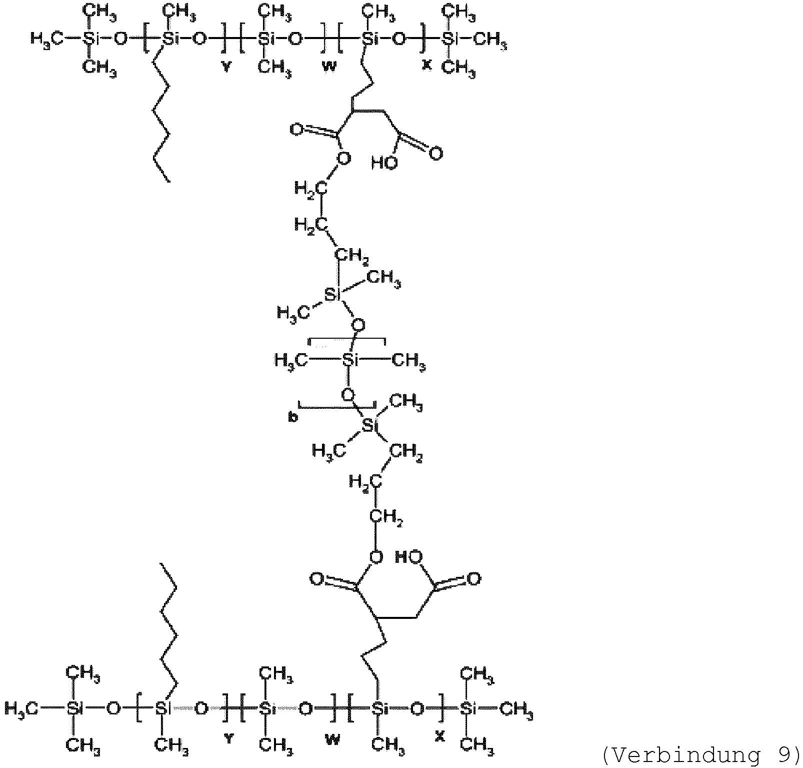

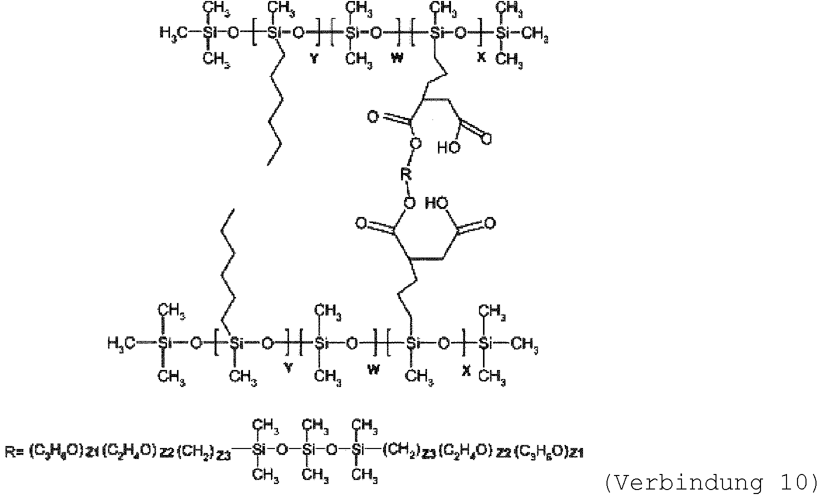

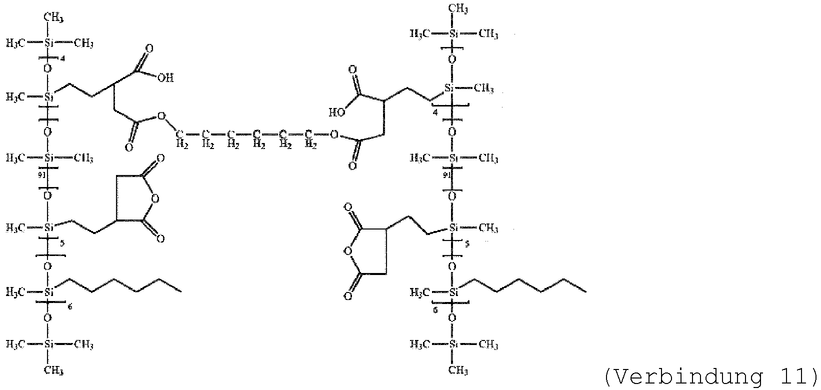

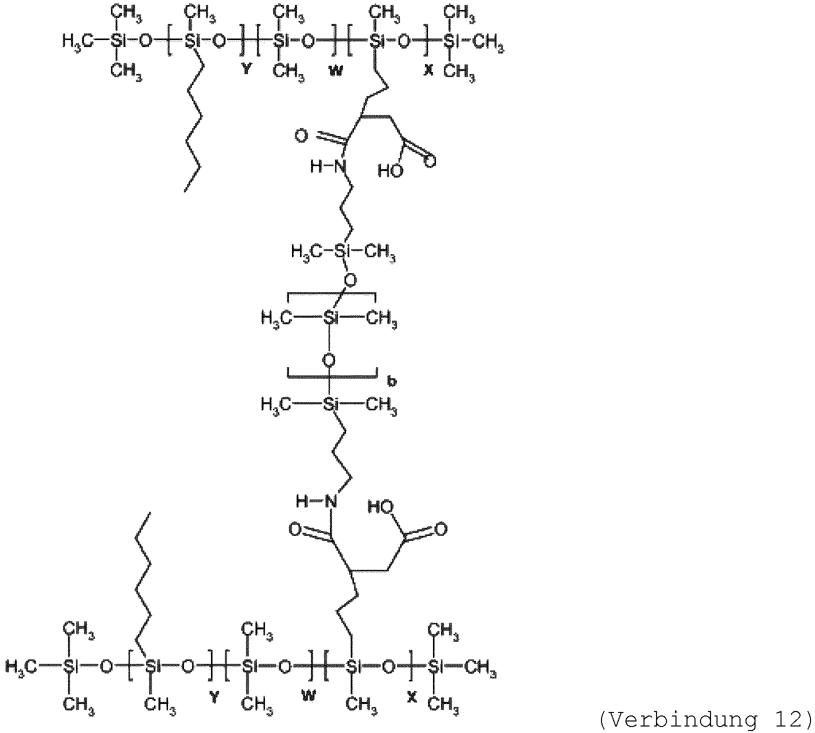

6. Zusammensetzung nach Anspruch 5, wobei es sich bei dem Silikonelastomer mit Carbonsäurefunktionen um Verbindung 11 handelt.

7. Zusammensetzung nach einem der Ansprüche 1 bis 3, wobei es sich bei dem Silikonelastomer mit Carbonsäurefunktionen um dasjenige mit dem folgenden INCI-Namen handelt: HEXYL/SUCCINYL DIMETHICONE CROSSPOLYMER.

8. Zusammensetzung nach einem der vorhergehenden Ansprüche, wobei das Silikonelastomer mit Carbonsäurefunktionen in Dispersion in mindestens einem Öl und in Gelform vorliegt; vorzugsweise ausgewählt aus flüchtigen Kohlenwasserstoffölen, flüchtigen Silikonölen, nichtflüchtigen Kohlenwasserstoffölen, nichtflüchtigen Silikonölen und Mischungen davon.

9. Zusammensetzung nach Anspruch 8, wobei die ölige Dispersion von Silikonelastomer mit Carbonsäurefunktionen 10 bis 40 Gew.-% Silikonelastomer-Aktivsubstanz und weiter bevorzugt 20 bis 35 Gew.-%, bezogen auf das Gesamtgewicht der öligen Dispersion, umfasst.

10. Zusammensetzung nach Anspruch 8 oder 9, wobei das Silikonelastomer mit Carbonsäurefunktionen in Dispersion in Isododecan und in Gelform vorliegt.

11. Zusammensetzung nach Anspruch 10, wobei das Silikonelastomer mit Carbonsäurefunktionen aus den Verbindungen 1, 4 und 11 gemäß Anspruch 4, vorzugsweise Verbindung 11, ausgewählt ist und insbesondere Verbindung 11 in der Dispersion auf Basis von Isododecan mit einem Aktivsubstanzgehalt von 30 Gew.-% vorliegt.

12. Zusammensetzung nach Anspruch 9, umfassend mindestens ein Silikonelastomer in Gelform in Dispersion in Isododecan mit dem INCI-Namen:
ISODODECANE (and) HEXYL/SUCCINYL DIMETHICONE CROSSPOLYMER, und spezieller mit einem Aktivsubstanzgehalt von 30 Gew.-% in der Dispersion auf Isododecan-Basis.

13. Zusammensetzung nach einem der vorhergehenden Ansprüche, wobei das Copolymer bzw. die Copolymere auf Basis von Silikonharz und von Silikon vom Dimethiconol-Typ in einem Gehalt von mehr als 1 Gew.-% und bis zu 40 Gew.-%, bezogen auf das Gesamtgewicht der Zusammensetzung, vorzugsweise im Bereich von 1,5 bis 20 Gew.-% und weiter bevorzugt im Bereich von 1,5 bis 15 Gew.-% vorliegt bzw. vorliegen.

14. Zusammensetzung nach einem der vorhergehenden Ansprüche, wobei das Copolymer auf Basis von Silikonharz und von Silikon vom Dimethiconol-Typ aus denjenigen mit dem folgenden INCI-Namen ausgewählt ist: TRIMETHYLSILOXYSILICATE/DIMETHICONOL CROSSPOLYMER.

15. Zusammensetzung nach einem der vorhergehenden Ansprüche, zusätzlich umfassend mindestens einen C₂-C₆-Monoalkohol, der insbesondere aus Isopropanol, Ethanol und Mischungen davon ausgewählt ist.

16. Zusammensetzung nach Anspruch 15, wobei die Menge an Monoalkohol(en) von 0,1 bis 10 Gew.-%, vorzugsweise von 1 bis 5 Gew.-%, bezogen auf das Gesamtgewicht der Zusammensetzung, variiert.

17. Zusammensetzung nach einem der vorhergehenden Ansprüche, zusätzlich umfassend mindestens ein Pigment, das vorzugsweise aus Eisenoxiden, Titandioxiden und Mischungen davon ausgewählt ist.

18. Zusammensetzung nach Anspruch 17, wobei das Pigment mit mindestens einer hydrophoben oder lipophilen Verbindung beschichtet ist, die insbesondere aus Silikonoberflächenmitteln; fluorierten Oberflächenmitteln; Fluorsilikonoberflächenmitteln; Metallseifen; N-acylierten Aminosäuren oder Salzen davon; Lecithin und Derivaten davon; Isopropyltriisostearyltitanat; Isostearylsebacat; natürlichen pflanzlichen oder tierischen Wachsen; polaren synthetischen Wachsen; Fettestern; Phospholipiden und Mischungen davon ausgewählt ist, weiter bevorzugt aus Fluorsilikonoberflächenmitteln ausgewählt ist und noch spezieller aus Perfluoralkyltrialkoxysilanen ausgewählt ist.

19. Zusammensetzung nach Anspruch 17 oder 18, enthaltend mindestens 5 Gew.-% Pigment(e), weiter bevorzugt 5 bis 40 Gew.-% Pigment(e), insbesondere 10 bis 30 Gew.-% und spezieller 10 bis 20 Gew.-% Pigment(e), bezogen auf das Gesamtgewicht der Zusammensetzung.

20. Verfahren zum Beschichten von Keratinmaterialien, insbesondere zum Schminken und/oder Pflegen von Keratinmaterialien, wie der Haut, **dadurch gekennzeichnet, dass** es das Aufbringen einer Zusammensetzung gemäß einem der vorhergehenden Ansprüche auf die Keratinmaterialien umfasst.

## Revendications

1. Composition d'entretien et/ou de maquillage de matières kératiniques, en particulier de la peau, comprenant, de préférence dans un milieu physiologiquement acceptable :
a) au moins un élastomère de silicone contenant des fonctions acide carboxylique ; et
b) au moins un copolymère à base de résine silicone et de silicone de type diméthiconol.

2. Composition selon la revendication 1, **caractérisée en ce qu'**elle est anhydre.

3. Composition selon la revendication 1 ou 2, comprenant une teneur en matière active d'élastomère de silicone contenant des fonctions acide carboxylique allant de 2 à 25 % en poids, de préférence allant de 5 à 15 % en poids par rapport au poids de la composition.

4. Composition selon l'une quelconque des revendications précédentes, dans laquelle l'élastomère de silicone contenant des fonctions acide carboxylique est choisi parmi ceux répondant à la formule (I) ci-dessous : 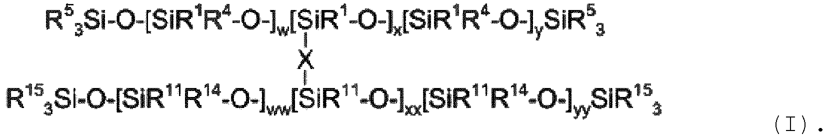 dans laquelle
le radical X correspond à la formule (i) ci-dessous 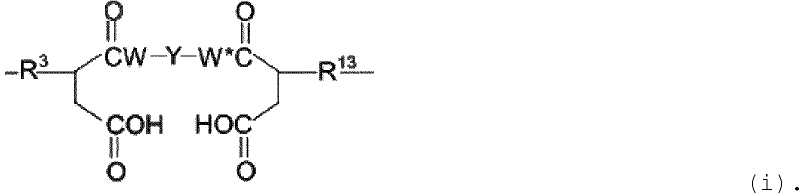 ou à la formule (I') ci-dessous : 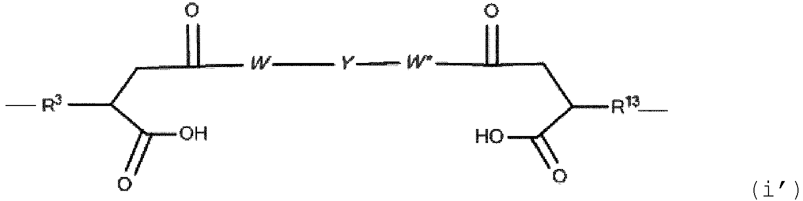
dans laquelle
W et W* désignent indépendamment un atome d'oxygène (O) ou un groupe N-R où chaque radical R désigne indépendamment un atome d'hydrogène (H) ou un radical R¹ ;
Y est un groupe divalent ;
R¹, R¹¹, R⁴, R¹⁴, R⁵ et R¹⁵ désignent indépendamment un groupe hydrocarbyle substitué ou non substitué ;
R³ et R¹³ désignent indépendamment un groupe divalent ;
w et ww sont, indépendamment, des entiers allant de 0 à 1 000 ;
x et xx sont, indépendamment, des entiers allant de 1 à 100 ;
y et yy sont, indépendamment, des entiers allant de 0 à 1 000 ;
de préférence, w et y ne sont pas simultanément égaux à 0 et ww et yy ne sont pas simultanément égaux à 0.

5. Composition selon l'une quelconque des revendications précédentes, dans laquelle l'élastomère de silicone de formule (I) est choisi parmi les composés 1 à 12 ci-dessous :

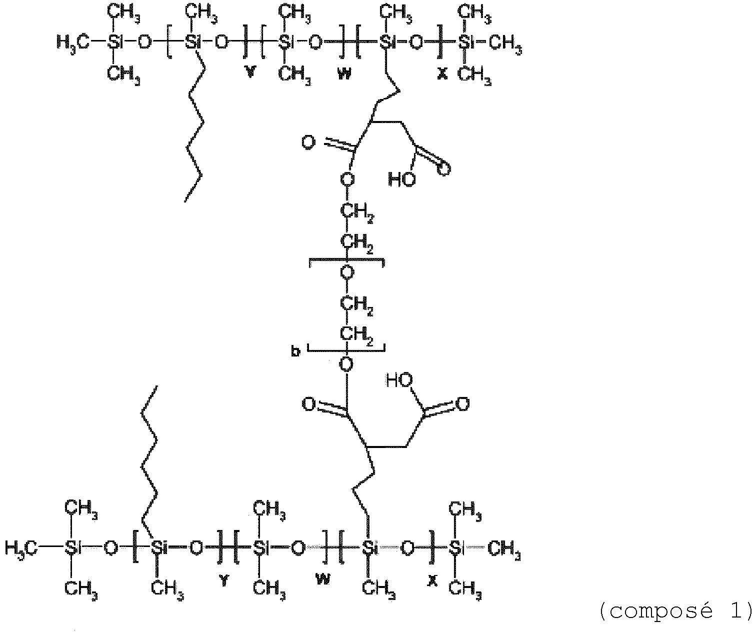

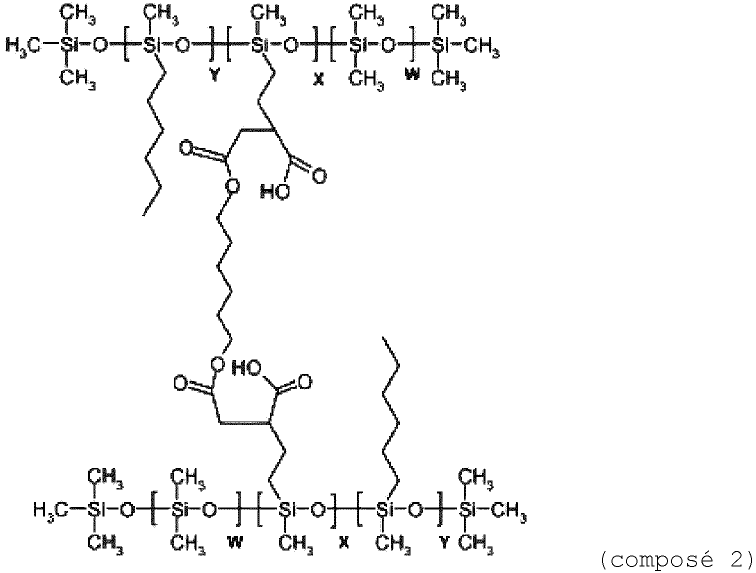

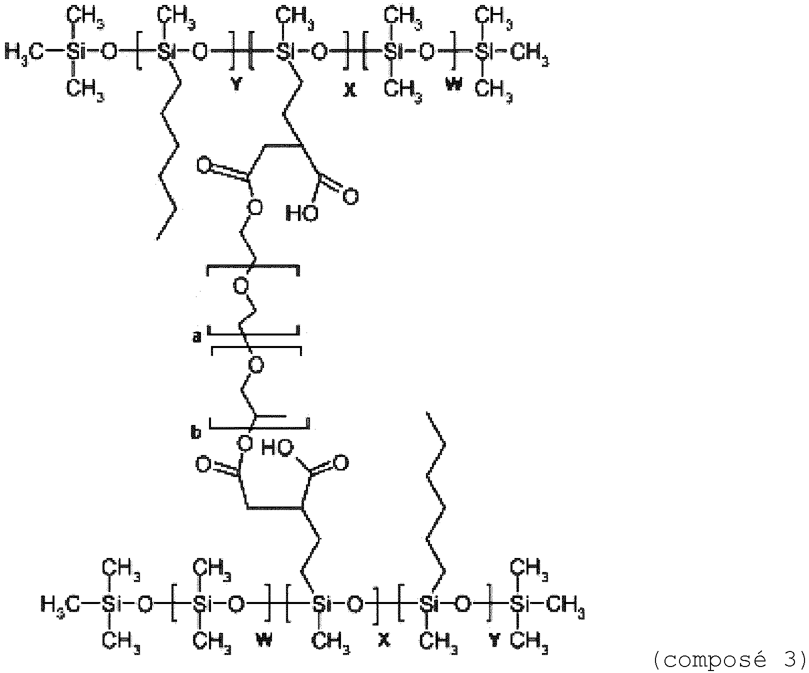

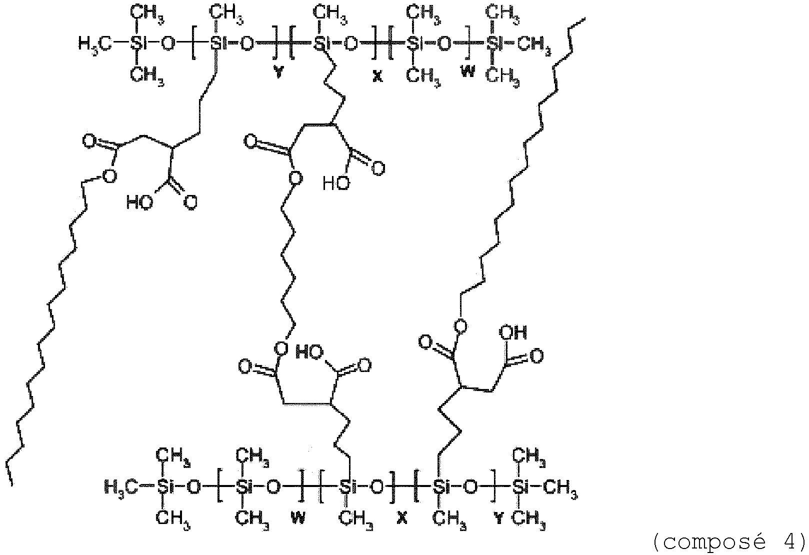

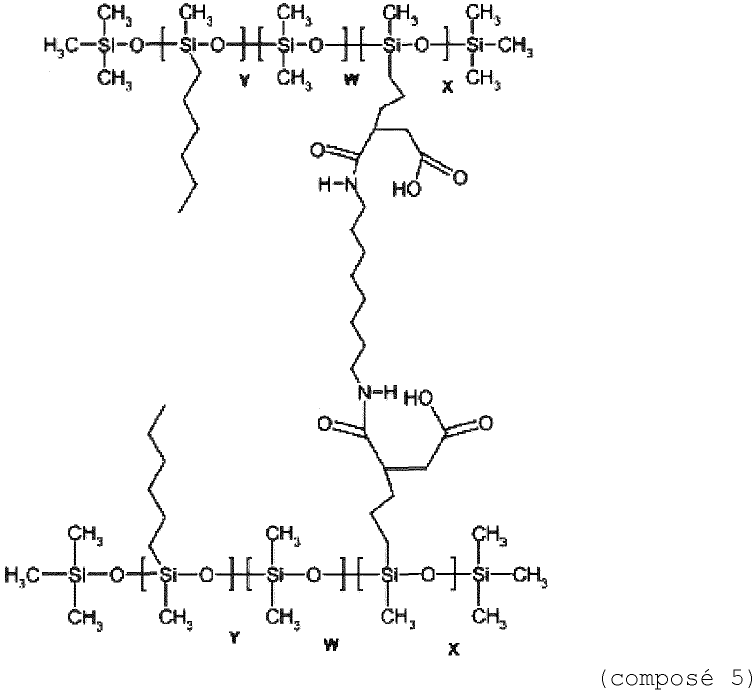

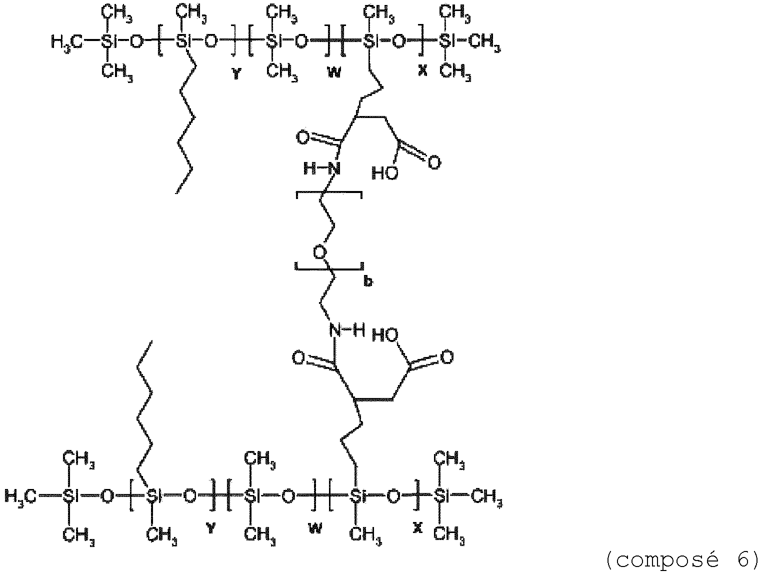

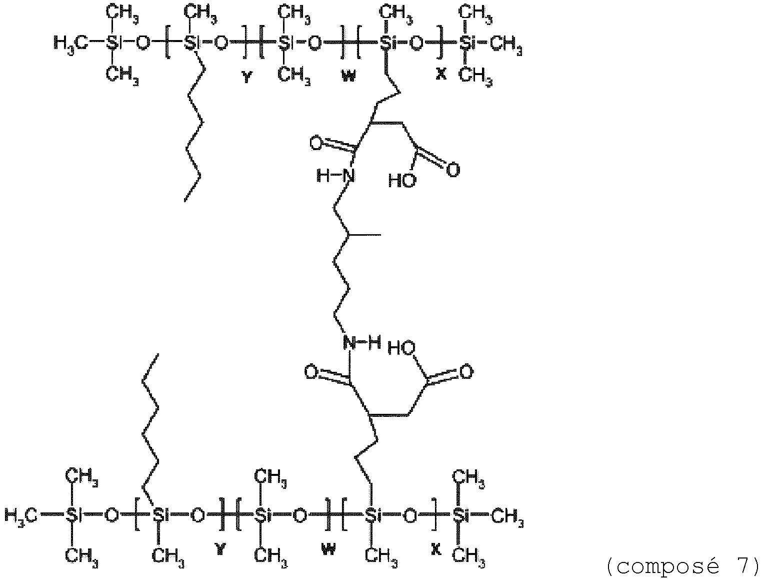

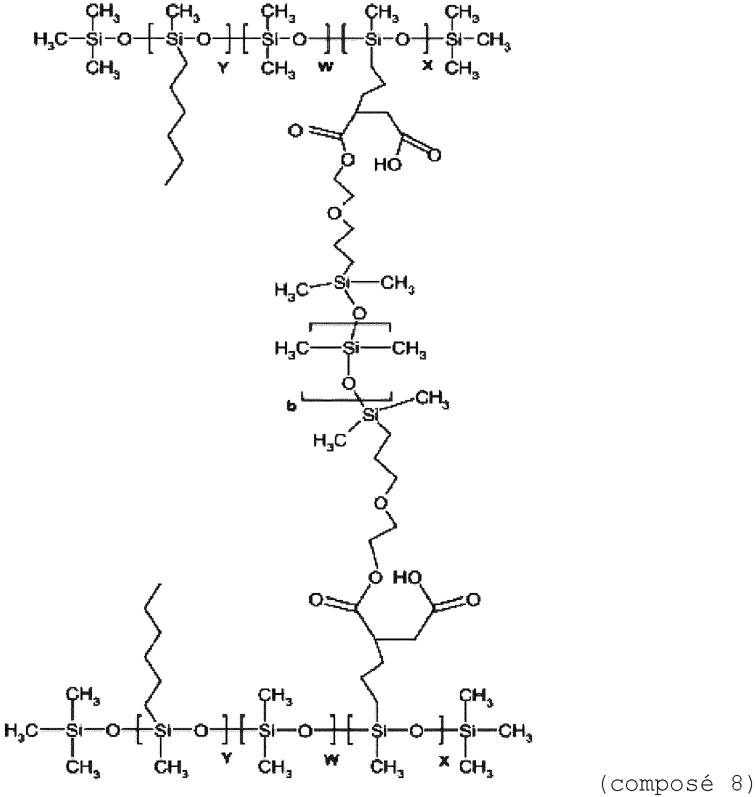

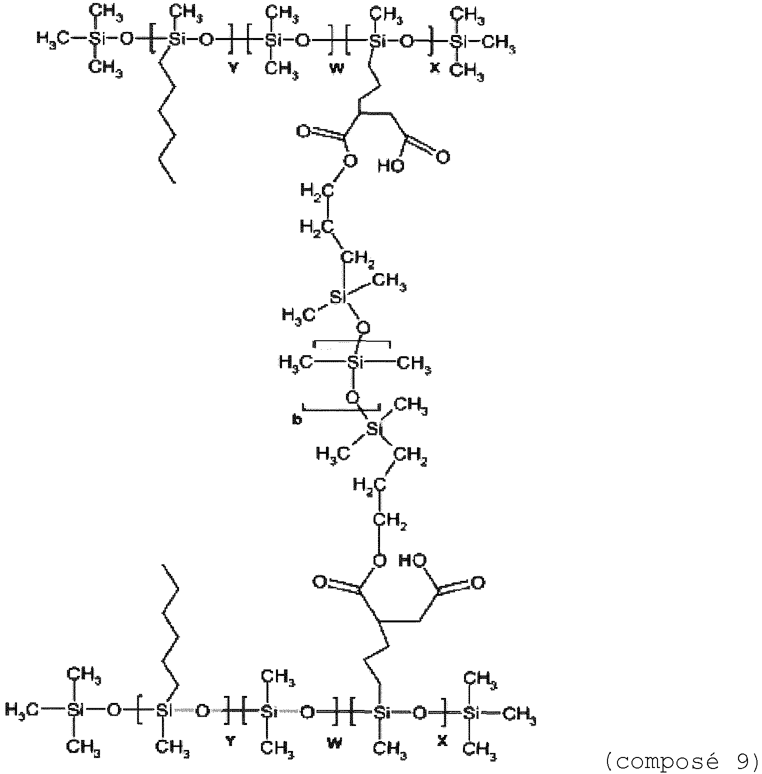

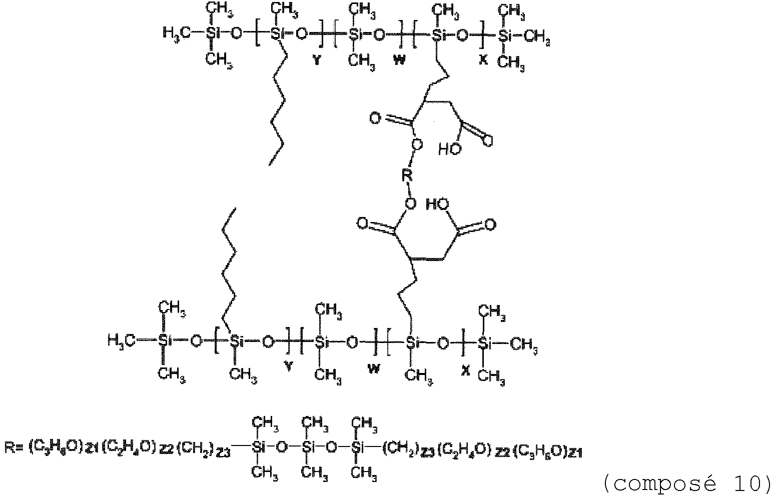

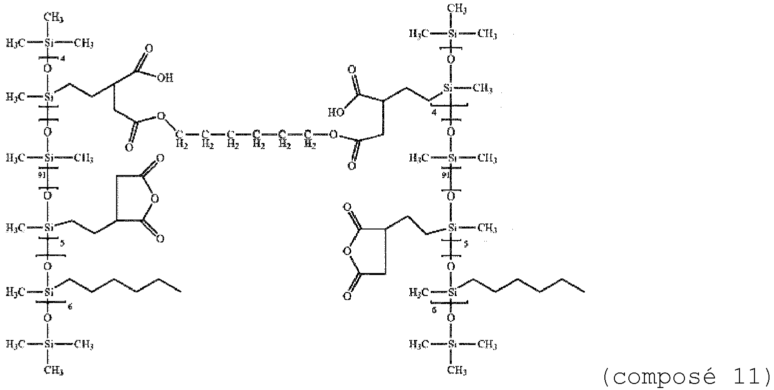

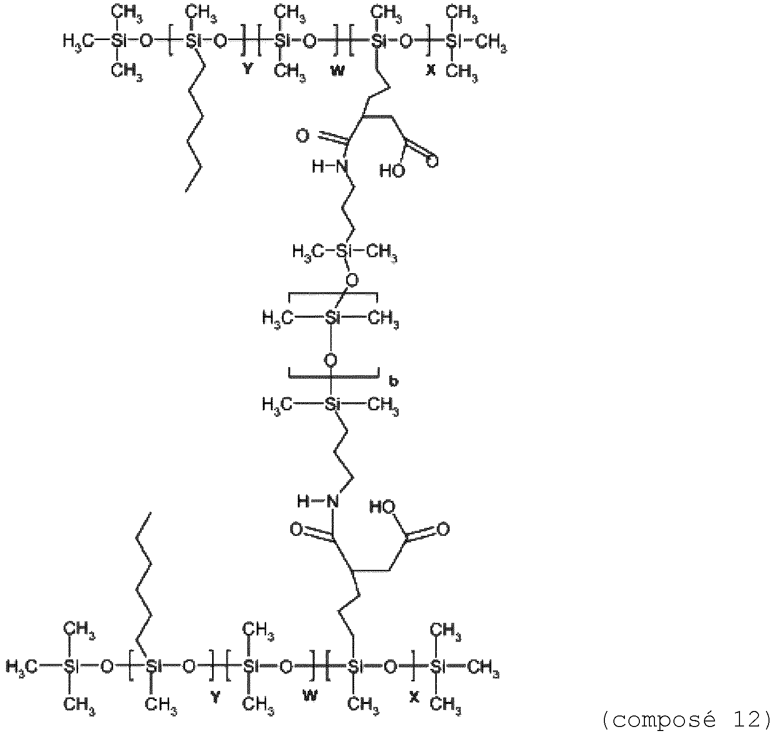

6. Composition selon la revendication 5, dans laquelle l'élastomère de silicone contenant des fonctions acide carboxylique est le composé 11.

7. Composition selon l'une quelconque des revendications 1 à 3, dans laquelle l'élastomère de silicone contenant des fonctions acide carboxylique est celui comportant la dénomination INCI : HEXYL/SUCCINYL DIMETHICONE CROSSPOLYMER.

8. Composition selon l'une quelconque des revendications précédentes, dans laquelle l'élastomère de silicone contenant des fonctions acide carboxylique est en dispersion dans au moins une huile et sous forme de gel ; de préférence choisi parmi les huiles d'hydrocarbure volatiles, les huiles de silicone volatiles, les huiles d'hydrocarbure non volatiles, les huiles de silicone non volatiles et leurs mélanges.

9. Composition selon la revendication 8, dans laquelle la dispersion huileuse d'élastomère de silicone contenant des fonctions acide carboxylique comprend de 10 à 40 % en poids de matière active élastomère de silicone, et plus préférablement de 20 à 35 % en poids par rapport au poids total de la dispersion huileuse.

10. Composition selon la revendication 8 ou 9, dans laquelle l'élastomère de silicone contenant des fonctions acide carboxylique est en dispersion dans de l'isododécane et sous forme de gel.

11. Composition selon la revendication 10, dans laquelle l'élastomère de silicone contenant des fonctions acide carboxylique est choisi parmi les composés 1, 4 et 11, tels que définis à la revendication 4, de préférence le composé 11, et plus particulièrement le composé 11 est présent dans la dispersion à base d'isododécane ayant une teneur en matière active de 30 % en poids.

12. Composition selon la revendication 9, comprenant au moins un élastomère de silicone sous forme de gel en dispersion dans l'isododécane ayant la dénomination INCI :
ISODODECANE (and) HEXYL/SUCCINYL DIMETHICONE CROSSPOLYMER, et plus particulièrement ayant une teneur en matière active de 30 % en poids dans la dispersion à base d'isododécane.

13. Composition selon l'une quelconque des revendications précédentes, dans laquelle le ou les copolymère (s) à base de résine silicone et de silicone de type diméthiconol sont présents en une teneur supérieure à 1 % et allant jusqu'à 40 % en poids par rapport au poids total de la composition, de préférence allant de 1,5 % à 20 % en poids, et plus préférablement allant de 1,5 % à 15 % en poids.

14. Composition selon l'une quelconque des revendications précédentes, dans laquelle le copolymère à base de résine silicone et de silicone de type diméthiconol est choisi parmi ceux comportant la dénomination INCI :
TRIMETHYLSILOXYSILICATE/DIMETHICONOL CROSSPOLYMER.

15. Composition selon l'une quelconque des revendications précédentes, comprenant en outre au moins un monoalcool en C₂-C₆, en particulier choisi parmi l'isopropanol, l'éthanol et leurs mélanges.

16. Composition selon la revendication 15, dans laquelle la quantité de monoalcool(s) varie de 0,1 % à 10 % en poids, de préférence de 1 % à 5 % en poids par rapport au poids total de ladite composition.

17. Composition selon l'une quelconque des revendications précédentes, comprenant en outre au moins un pigment, de préférence choisi parmi les oxydes de fer, les dioxydes de titane et leurs mélanges.

18. Composition selon la revendication 17, dans laquelle le pigment est revêtu par au moins un composé hydrophobe ou lipophile, en particulier choisi parmi les agents de surface de silicone ; les agents de surface fluorés ; les agents de surface fluorosiliconés ; les savons métalliques ; les acides aminés N-acylés ou leurs sels ; la lécithine et ses dérivés ; le titanate d'isopropyle triisostéaryle ; le sébaçate d'isostéaryle ; les cires naturelles végétales ou animales ; les cires synthétiques polaires ; les esters gras ; les phospholipides ; et leurs mélanges, plus préférablement choisis parmi les agents de surface fluorosiliconés, encore plus particulièrement choisis parmi les perfluoroalkyltrialcoxysilanes.

19. Composition selon la revendication 17 ou 18, comprenant au moins 5 % en poids de pigment (s), plus préférablement de 5 % à 40 % en poids de pigment(s), en particulier de 10 % à 30 % en poids, et plus particulièrement de 10 % à 20 % en poids de pigment(s), par rapport au poids total de ladite composition.

20. Procédé de revêtement de matières kératiniques, plus particulièrement de maquillage et/ou d'entretien de matières kératiniques telles que la peau, **caractérisé en ce qu'**il comprend l'application sur les matières kératiniques d'une composition telle que définie dans l'une quelconque des revendications précédentes.
